# FASCICULE DE BREVET EUROPEEN

(11) **EP 0 896 620 B1**
(45) Date de publication et mention de la délivrance du brevet: **03.08.2005**
(21) Numéro de dépôt: 97906232.0
(22) Date de dépôt: 20.02.1997
(51) Int. Cl.: C12N 15/12, C07K 14/72, C12N 15/62, A01K 67/027, A61K 38/16, C12N 15/90, C12N 9/00

(54) **RECEPTEUR NUCLEAIRE DE GLUCOCORTICOIDES MODIFIE, PROTEINE DE FUSION, FRAGMENTS D'ADN CODANT POUR LEDIT RECEPTEUR ET LADITE PROTEINE DE FUSION**
MODIFIZIERTEN GLUCOCORTICOID REZEPTOR, FUSIONSPROTEINEN, UND DNA SOWIE DIESE REZEPTORUND FUSIONSPROTEINEN KODIERENDEN
MODIFIED NUCLEAR GLUCOCORTICOID RECEPTOR, FUSION PROTEIN, AND DNA FRAGMENTS CODING FOR SAID RECEPTOR AND SAID FUSION PROTEIN

(30) Priorité: 20.02.1996 FR 9602060
(43) Date de publication de la demande: 17.02.1999
(73) Titulaire: ASSOCIATION POUR LE DEVELOPPEMENT DE LA RECHERCHE EN GENETIQUE MOLECULAIRE, (A.DE.RE.GE.M), 75001 Paris (FR)
(72) Inventeur: BROCARD, Jacques, Bertrand, F-67000 Strasbourg (FR); CHAMBON, Pierre, Henri, F-67113 Blaesheim (FR); GRONEMEYER, Hinrich, D-77704 Oberkirch (DE); METZGER, Daniel, F-67100 Strasbourg (FR); NICOLAS, Jean-Claude, F-34090 Montpellier (FR); ROUX, Sylvie, F-34074 Montpellier (FR)
(74) Mandataire: Ahner, Francis
(86) Numéro de dépôt international: PCT/FR1997/000315
(87) Numéro de publication internationale: WO 1997/031108

(56) Documents cités:
- WO-A-88/03168
- WO-A-90/07517
- US-A- 5 215 916
- US-A- 5 468 624
- J. BIOL. CHEM., vol. 269, no. 46, 18 Novembre 1994, pages 29010-29015, XP000611464 N. WARRIAR ET AL.: "Hormone binding domain of human glucocorticoid receptor." cité dans la demande
- J. BIOL. CHEM., vol. 266, no. 33, 25 Novembre 1991, pages 22075-22078, XP000611463 P.K. CHAKRABORTI ET AL.: "Creation of "Super" Glucocorticoid receptors by point mutations in the steroid binding domain." cité dans la demande
- MOLECULAR AND CELLULAR BIOLOGY, vol. 15, no. 2, Février 1995, pages 1005-1013, XP000564501 W. LIU ET AL.: "Hormone-independent repression of AP-1-Inducible collagenase promoter activity by Glucocorticoid receptors."
- J. BIOL. CHEM., vol. 269, no. 11, 18 Mars 1994, pages 7914-7918, XP002011282 D. CHEN ET AL.: "The hormone-binding role of 2 cysteines near the C terminus of the mouse glucocorticoid receptor." cité dans la demande
- NATURAL STRUCURAL BIOLOGY, vol. 3, 1 Janvier 1996, pages 87-94, XP000610199 J-M. WURTZ ET AL.: "A canonical structure for the ligand-binding domain of nuclear receptors." cité dans la demande
- JOURNAL OF STEROID BIOCHEMISTRY AND MOLECULAR BIOLOGY 55 (2). 1995. 135-146. ISSN: 0960-0760, XP000610413 JEWELL C M ET AL: "Immunocytochemical analysis of hormone mediated nuclear translocation of wild type and mutant glucocorticoid receptors."
- AMERICAN JOURNAL OF RESPIRATORY CELL AND MOLECULAR BIOLOGY 11 (1). 1994. 42-48. ISSN: 1044-1549, XP000610412 LANE S J ET AL: "Chemical mutational analysis of the human glucocorticoid receptor cDNA in glucocorticoid -resistant bronchial asthma."
- J. CLIN. INVEST., vol. 87, no. 2, Février 1991, pages 680-686, XP000610523 HURLEY ET AL.: "Point mutation causing a single amino acid substitution in the hormone binding domain of the gucocorticoid receptor in familial glucocorticoid resistance." cité dans la demande
- J. CLIN. INVEST., vol. 91, no. 5, Mai 1993, pages 1918-1925, XP000610522 D.M. MALCHOFF ET AL.: "A mutation of the glucocorticoid receptor in primary cortisol resistance." cité dans la demande

## Description

La présente invention concerne un fragment d'ADN codant pour un récepteur nucléaire de glucocorticoïdes (GR) modifié, et un fragment d'ADN codant pour un domaine de liaison du ligand (LBD) dudit récepteur, ainsi qu'un fragment d'ADN codant pour une protéine de fusion comportant ledit récepteur ou ledit domaine de liaison du ligand dudit récepteur fusionné à une protéine dont l'activité est induite en présence de ligands glucocorticoïdes.

La présente invention concerne également un récepteur nucléaire de glucocorticoïdes modifié, notamment le récepteur humain et son domaine de liaison du ligand modifié, ainsi qu'une protéine de fusion comportant ledit récepteur ou ledit domaine de liaison du ligand.

La présente invention concerne également un vecteur d'expression conditionnelle d'une protéine, notamment une protéine étrangère dans des cellules hôtes humaines ou animales, notamment des cellules de mammifères. La présente invention se rapporte aussi à un procédé d'expression d'une protéine dans lesdites cellules. Par ailleurs, la présente invention concerne un procédé d'excision, insertion, inversion, ou translocation conditionnelle d'un fragment d'ADN dans des cellules hôtes humaines ou animales, notamment de mammifères.

La présente invention concerne en outre un vecteur de transfert d'un fragment d'ADN dans lesdites cellules hôtes humaines ou animales, notamment de mammifères, et son utilisation comme médicament, comme outil d'analyse et étude du fonctionnement d'un gène ainsi qu'une méthode de traitement de cellules ex vivo ou in vitro.

Enfin, la présente invention concerne les cellules humaines ou animales transfectées par un vecteur d'expression et/ou de transfert selon l'invention ainsi que les animaux transgéniques qui en dérivent.

Le récepteur des glucocorticoïdes (GR) est une protéine régulatrice de transcription de gènes qui médie l'action des glucocorticoïdes dans les cellules cibles. Le GR et d'autres membres de la superfamille des récepteurs nucléaires (facteurs de transcription activés par des ligands) présentent une structure modulaire commune. La région N-terminale variable contient l'activité de transactivation constitutive AF-1. Le domaine central de liaison à l'ADN (DBD) est hautement conservé entre les différentes espèces et permet la liaison du récepteur à son élément de réponse ADN apparenté (GRE dans le cas du GR). Le domaine de liaison du ligand (LBD), localisé dans la région C-terminale du GR, non seulement fixe le ligand, mais également comporte de multiples activités distinctes : une surface pour l'interaction avec hsp90 et une surface distincte pour l'homodimérisation, un signal de localisation nucléaire et une fonction de transactivation AF-2 dépendant du ligand (pour les articles de synthèse et références, voir (1-5)). Le domaine LBD est fortement conservé entre les différentes espèces. Une fonction de transactivation AF-2 a été identifié dans la partie C-terminale de divers LBD (6-10). L'intégrité de la partie centrale de la région contenant AF-2 est requise pour l'activation de transcription, dépendante de ligand, par les récepteurs nucléaires correspondants, et pour l'interaction, entre cette région et des facteurs intermédiaires transcriptionnels apparentés (TIF, également dénommés co-activateurs ou médiateurs) (11-19). Il a été proposé un modèle général (19) dans lequel la liaison du ligand induit un changement conformationnel du domaine de liaison du ligand en faisant intervenir en particulier la région C-terminale hébergeant la partie centrale de AF-2, en engendrant ainsi une surface pour une interaction efficace avec les TIF. Les LBD des récepteurs nucléaires contiennent des régions conservées possédant une structure canonique en hélice. Ces données structurales ont permis d'effectuer un alignement commun pour les LBD de tous les récepteurs nucléaires. L'hélice H12 contient la fonction de transactivation AF-2 (19). Les acides aminés des hélices H11 et H12, notamment des récepteurs des glucocorticoïdes de différentes espèces telles que l'homme, le rat, la souris et le xénope, sont conservés.

Plusieurs mutations dans le LBD de GR ont été décrites précédemment. On peut distinguer deux types majeurs de mutations :
- le premier type consiste en mutations qui affectent positivement ou négativement l'affinité de liaison au ligand. Par exemple, les remplacements du résidu cystéine 656 par la glycine dans le GR de rat (20) et du résidu méthionine 565 par l'arginine ou du résidu alanine 573 par la glutamine dans le GR humain (hGR)(21) augmentent l'affinité de liaison au ligand et conduisent à un décalage de la courbe de réponse en fonction de la dose pour la transactivation en direction des plus basses concentrations de ligand. Ces mutants sont par conséquent désignés par "super GR". De même, on a rapporté des mutants ayant une affinité de liaison au ligand plus faible (21-24). Les mutations de LBD qui décalent la courbe de réponse en fonction de la dose de ligand en direction des plus hautes concentrations de ligand, résultent d'une affinité altérée de liaison de l'hormone (25-26) ;
- le second groupe de mutants LBD comprend ceux affectant la fonction d'activation transcriptionnelle (AF-2), sans altérer la liaison au ligand. Comme exemples, on peut citer des mutations dans la région contenant AF-2 qui sont liées à une perte ou une diminution du potentiel de transactivation, mais n'affectent pas l'affinité de liaison au ligand (6-10).

La fusion du domaine de liaison du ligand (LBD) de récepteurs nucléaires à des protéines hétérologues a permis de contrôler, dans de nombreux cas, l'activité de ces dernières.

Les activités de Myc, c-Abl, Src, erbB1, Raf, E1A Cre et FLP, peuvent être modulées en réalisant des protéines de fusion avec le LBD de récepteurs nucléaires (27-29). Cependant, les ligands de ces récepteurs sont présents dans de nombreux systèmes biologiques, pouvant ainsi induire un niveau d'activité basal. Pour éviter de tels problèmes, un LBD muté du récepteur des oestrogènes (RE), a été fusionné à la protéine c-Myc (30), ou aux recombinases Cre et FLP (28 et 29).

Les recombinases de la famille des intégrases λ catalysent l'excision, l'insertion, l'inversion ou la translocation de fragments d'ADN au niveau de sites spécifiques de reconnaissance desdites recombinases (31-36). Les recombinases sont actives dans les cellules animales (35).

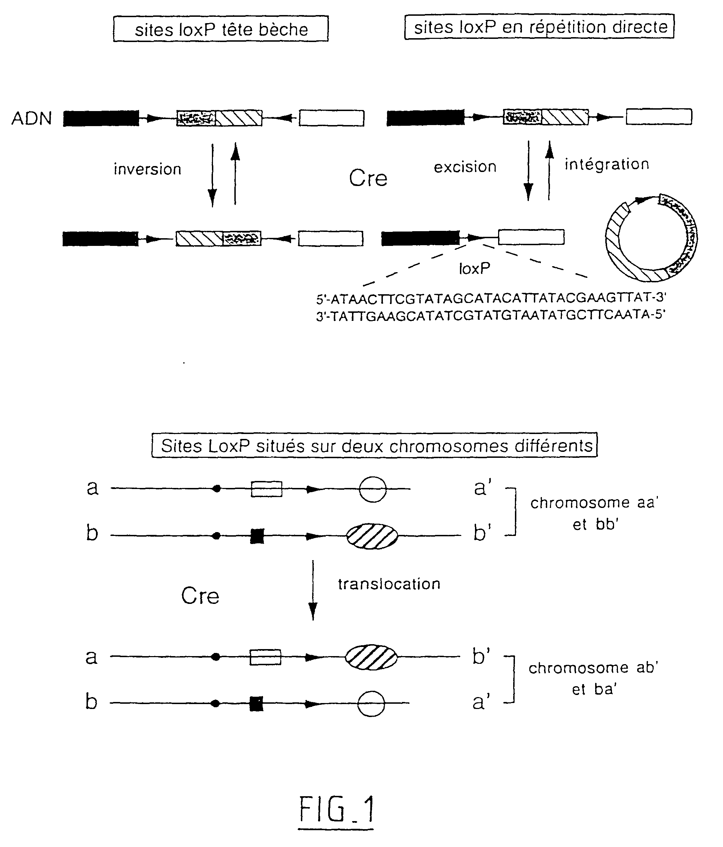

La recombinase Cre, une intégrase de 38 KDa du bactériophage P1, catalyse la recombinaison entre deux séquences d'ADN de 34 paires de bases appelées loxP en l'absence de cofacteurs (32, Figure 1).

La position sur une ou plusieurs molécules d'ADN et l'orientation de sites loxP l'un par rapport à l'autre déterminent le type de fonction de la recombinase, excision, insertion, inversion ou translocation notamment, la recombinase Cre (figure 1). Ainsi l'activité recombinase de Cre est une inversion lorsque deux sites LoxP sont tête-bêche sur un même fragment d'ADN et une excision, lorsque les sites LoxP sont en répétition directe sur un même fragment d'ADN. L'activité de la recombinase est une insertion lorsqu'un site loxP est présent sur un fragment d'ADN, une molécule d'ADN telle qu'un plasmide contenant un site loxP pouvant être insérée au niveau dudit site loxP (figure 1). La recombinase Cre peut également induire une translocation entre deux chromosomes à condition qu'un site loxP soit présent sur chacun d'eux (37) (figure 1). De manière plus générale, la recombinase Cre est donc capable d'induire la recombinaison entre une ou plusieurs molécules d'ADN différentes, à condition qu'elles soient porteuses de sites loxP.

De même la recombinase FLP, une recombinase de 43 KDa de Saccharomyes cerevisiae, est capable du même type d'action sur les fragments d'ADN contenant des sites de reconnaissance FRT (34).

En réalisant une protéine de fusion (chimère) entre la recombinase Cre et la région C-terminale du récepteur humain des oestrogènes contenant une Valine en position 400, on obtient une molécule capable d'exciser, en présence du ligand du récepteur, l'oestradiol, les séquences d'ADN localisées entre deux sites loxP, ainsi qu'un des sites loxP, lorsque ceux-ci sont en répétition directe, alors qu'en absence de ligand, l'excision n'a pas lieu (28).

L'activité de la recombinase FLP est également régulable, en réalisant des chimères entre la FLP et le domaine de liaison de récepteurs nucléaires. En fusionnant la FLP avec le LBD du récepteur des oestrogènes ou du récepteur des glucocorticoïdes en l'absence de ligand, l'activité recombinase est très faible, alors qu'elle est rapidement induite par les ligands respectifs des LBD (29).

Toutefois, l'utilisation de protéines de fusion entre des recombinases et des LBD de récepteurs nucléaires pour exciser de façon contrôlée des fragments d'ADN situés entre des sites de reconnaissance desdites recombinases peut poser problème, étant donné que les concentrations de ligand présentes chez l'animal ou dans les milieux de culture des cellules peuvent être suffisantes pour induire au moins partiellement, l'activité recombinase.

La présente invention fournit un système de recombinaison inductible chez les animaux, notamment les mammifères, grâce à l'utilisation d'une protéine de fusion réalisée entre une recombinase, et le domaine de liaison du ligand, dérivé du récepteur des glucocorticoïdes, dont l'activité est inductible par des glucocorticoïdes synthétiques, mais pas par des glucocorticoïdes naturels notamment à des concentrations inférieures à 10-6 M c'est-à-dire physiologiques.

Pour éviter l'induction de l'activité recombinase de la protéine chimère par les ligands endogènes, la présente invention se propose de fournir en effet une mutation dans le LBD du récepteur des glucocorticoïdes lui permettant d'être plus fortement activé en présence de ligands glucocorticoïdes synthéthiques, qu'en présence de ligands glucocorticoïdes naturels aux concentrations physiologiques.

La présente invention a en effet pour objet un récepteur nucléaire des glucocorticoïdes modifié, notamment muté dans la région du domaine de liaison du ligand, de sorte que l'activité dudit récepteur est inductible plus fortement par un ligand glucocorticoïde synthétique que par un ligand glucocorticoïde naturel.

Selon la présente invention on entend par "récepteur (muté) des glucocorticoïdes inductible plus fortement par un ligand glucocorticoïde synthétique que par un ligand glucocorticoïde naturel" que l'activité du récepteur muté selon l'invention est induite plus fortement par des ligands glucocorticoïdes synthétiques que par des ligands glucocorticoïdes naturels à une concentration donnée et que le récepteur muté des glucocorticoïdes selon l'invention est beaucoup moins sensible aux glucocorticoïdes naturels que le récepteur sauvage.

Par "activité dudit récepteur" on entend ici l'activité transcriptionnelle dudit récepteur et/ou le cas échéant l'activité de régulation de l'activité d'une protéine fusionnée audit récepteur ou audit domaine de liaison du ligand, induite en présence de glucocorticoïdes. On cite plus particulièrement l'activité d'une protéine recombinase fusionnée audit récepteur ou audit domaine de liaison du ligand dudit récepteur.

Par "glucocorticoïdes naturels", on entend ici des hormones stéroïdes sécrétées par le cortex des glandes surrenales telles que le cortisol, la corticostérone, ou l'aldostérone. Par "glucocorticoïdes synthétiques", on entend des agonistes de synthèse chimique dudit récepteur pour l'activité concernée. On cite parmi ceux-ci, la dexamethasone, le triamcinolone acetonide, le RU 486, le RU 28362, le bimedrazol, le fluocinolone acetonide.

Certains ligands synthétiques seront agonistes pour induire unedite activité particulière du récepteur et antagonistes par rapport à une autre activité du récepteur. Ainsi, le composé RU 486 induit l'activité d'une protéine recombinase fusionnée audit récepteur ou audit domaine de liaison du ligand du récepteur mais n'induit pas l'activité transcriptionnelle du récepteur.

Le ligand glucocorticoïde synthéthique peut se lier au récepteur nucléaire sauvage (non muté), mais il se peut que ledit ligand synthétique se lie au récepteur muté et pas au récepteur naturel. Il se peut aussi que si ledit ligand synthétique se lie au récepteur sauvage, l'activité de celui-ci ne soit néanmoins pas induite par liaison au ligand synthétique.

On a en particulier, identifié une mutation dans le domaine LBD des récepteurs nucléaires des glucocorticoïdes (isoleucine --> thréonine) située entre les hélices H11 et H12 (19). Cette mutation entraîne une perte d'activité du récepteur en présence de glucocorticoïdes naturels. Plus précisément, ce récepteur des glucocorticoïdes muté entre H11 et H12 n'a pas ou très peu d'activité transcriptionnelle en présence de glucocorticoïdes naturels aux concentrations physiologiques naturelles de ceux-ci notamment des concentrations inférieures à 10-6 M. Son activité peut cependant être induite par des ligands glucocorticoïdes synthétiques, notamment la dexamethasone (dex).

La présente invention a plus particulièrement pour objet un récepteur humain muté appelé hGR (1747/T) caractérisé en ce qu'il a pour séquence, la séquence en acides aminés du récepteur nucléaire humain des glucocorticoïdes sauvage avec une mutation isoleucine --> thréonine en position 747, et plus particulièrement encore un récepteur nucléaire humain modifié qui a pour séquence en acides aminés, une séquence substantiellement telle que représentée dans l'IDS n° 1.

La mutation de l'isoleucine 747 en thréonine dans la partie C-terminale du domaine de liaison du ligand (LBD) du récepteur des glucocorticoïdes altère la capacité des ligands naturels à en induire l'activité de transactivation. Les glucocorticoïdes naturels tels que le cortisol, l'aldostérone ou la corticostérone sont très faiblement actifs ou totalement inactifs avec le mutant GR (I747/T), tandis que des glucocorticoïdes synthétiques tels que la dexaméthasone (Dex) stimulent efficacement la transactivation médiée par GR (I747/T). Toutefois, la courbe correspondante de réponse en fonction de la dose de ligand, pour la transactivation induite par Dex, est décalée vers les plus fortes concentrations, par comparaison avec celle obtenue avec le GR de type sauvage (TS). Ni le décalage, ni l'inaptitude du cortisol à activer efficacement GR (I747/T) ne sont dûs à une affinité altérée de liaison au ligand in vitro.

En effet, la mutation dans le LBD de GR (1747/T) n'altère pas sensiblement l'affinité de liaison au ligand in vitro. Toutefois, ce mutant présente un important décalage par rapport au GR TS de la courbe d'activité vers les fortes concentrations de ligands. Aussi des concentrations dites physiologiques de ligands glucocorticoïdes naturels suffisantes pour activer le GR TS, n'entraînent qu'une activité résiduelle ou nulle chez le GR mutant.

Ce mutant diffère des autres mutants de LBD de GR décrits précédemment (22, 38), dont la fonction d'activation altérée est en étroite corrélation avec une modification de leurs propriétés de liaison au ligand in vitro.

La présente invention a aussi pour objet un domaine de liaison du ligand du récepteur nucléaire de glucocorticoïdes selon l'invention et particulièrement, le domaine LBD [GR(I747/T)] caractérisé en ce qu'il a une mutation isoleucine --> thréonine entre les hélices H11 et H12. Plus particulièrement il a pour séquence, la séquence en acides aminés du domaine de liaison du ligand du récepteur des glucocorticoïdes humain substantiellement telle que représentée dans l'IDS n° 1 de l'acide aminé 532 à l'acide aminé 777 avec une mutation isoleucine --> thréonine en position 747.

La présente invention fournit également un fragment d'ADN codant pour un récepteur nucléaire de glucocorticoïdes modifié, notamment muté dans la région du domaine de liaison du ligand (LBD) selon l'invention, ainsi qu'un fragment d'ADN codant pour un domaine de liaison du ligand (LBD) du récepteur modifié selon l'invention.

La présente invention a donc plus précisément pour objet un fragment d'ADN codant pour un récepteur nucléaire des glucocorticoïdes sauvage avec une mutation de l'isoleucine en thréonine située entre les hélices H11 et H12 de la séquence d'acides aminés dudit récepteur, et plus particulièrement encore un fragment d'ADN caractérisé en ce qu'il a pour séquence, une séquence codante du récepteur nucléaire humain des glucocorticoïdes dont la séquence en acides aminés est substantiellement telle que représentée dans l'IDS n°1.

Dans un mode de réalisation, la présente invention a pour objet un fragment d'ADN, caractérisé en ce qu'il a pour séquence, la séquence de l'ADNc du récepteur nucléaire humain des glucocorticoïdes substantiellement telle que représentée dans l'IDS n° 1 comportant le codon ACC codant pour la thréonine en position 747 de la séquence d'acides aminés dudit récepteur.

De même la présente invention a pour objet un fragment d'ADN codant pour le domaine de liaison du ligand (LBD) du récepteur nucléaire des glucocorticoïdes modifié avec une mutation isoleucine --> thréonine située entre les hélices H11 et H12 et notamment en position correspondant à la position 747 de la séquence en acides aminés du récepteur nucléaire humain des glucocorticoïdes.

Plus particulièrement, la présente invention fournit un fragment d'ADN codant pour le domaine de liaison du ligand du récepteur nucléaire humain des glucocorticoïdes modifié, caractérisé en ce qu'il a pour séquence, une séquence codant pour les acides aminés du domaine de liaison du ligand du récepteur nucléaire humain des glucocorticoïdes dont la séquence en acides aminés est substantiellement telle que représentée dans l'IDS n° 1 de l'acide aminé 532 à l'acide aminé 777.

Plus particulièrement encore, la présente invention fournit un fragment d'ADN, caractérisé en ce qu'il a pour séquence, la séquence d'ADNc codant pour le domaine de liaison du ligand (LBD) du récepteur nucléaire humain des glucocorticoïdes substantiellement telle que représentée dans l'IDS n° 1 du codon 532 au codon 777 avec le codon ACC en position 747.

La présente invention a en outre pour objet un système vectoriel d'expression conditionnelle d'une protéine notamment une protéine étrangère dans des cellules hôtes comportant un élément de contrôle de la transcription inductible par un complexe formé par un récepteur nucléaire des glucocorticoïdes et un ligand, caractérisé en ce qu'il comprend :
- un premier fragment d'ADN consistant en un fragment d'ADN codant pour ladite protéine sous le contrôle d'éléments assurant son expression dans lesdites cellules hôtes, lesdits éléments assurant son expression comprenant une séquence de contrôle de transcription (RE) inductible par le récepteur selon l'invention complexé à un ligand glucocorticoïde synthétique, et
- un deuxième fragment d'ADN consistant en un fragment d'ADN fonctionnel codant pour ledit récepteur selon l'invention ou seulement une partie dudit fragment comprenant la région qui reconnaît le ligand (LBD) selon l'invention, et la région de liaison à l'ADN (DBD) qui se fixe sur ladite séquence de contrôle de la transcription (RE).
- lesdits premier et deuxième fragments d'ADN pouvant être portés par un même vecteur ou deux vecteurs séparément.

Dans un mode de réalisation, le système vectoriel comporte l'ADNc codant pour :
- la région LBD, représentée par des codons codant les acides aminés n° 532 à 777 dans l'IDS n° 1, et
- la région DBD, représentée par des codons codant les acides aminés n° 421 à 487 dans l'IDS n° 1.

Certaines cellules ont du GR endogène qui peut être activé par des ligands naturels et des ligands synthétiques. Si on veut activer un gène donné à volonté dans de telle cellules, sans qu'il puisse être activé par les ligands endogènes ni par les récepteurs endogènes, il faut que ce gène contienne un RE différent du GRE et utiliser un activateur chimérique comportant le DBD correspondant et un LBD muté selon l'invention. Ce gène pourra donc être activé par des ligands synthétiques, mais non par des ligands naturels.

C'est pourquoi dans un mode de réalisation, on remplace le domaine de liaison à l'ADN (DBD) du récepteur des glucocorticoïdes par celui d'un autre transactivateur, notamment celui de la protéine de levure Gal 4 et on remplace la séquence de contrôle de transcription (RE) du récepteur des glucocorticoïdes par celle d'un autre transactivateur, notamment la séquence 17 mère (17m) reconnue par la protéine Gal4.

La présente invention a donc également pour objet un procédé d'expression d'une protéine étrangère dans des cellules humaines ou animales notamment de mammifères, caractérisé en ce que l'on cultive des cellules qui contiennent un système vectoriel d'expression selon l'invention, et en ce qu'on ajoute ledit ligand synthétique, dans le milieu de culture, puis on récupère la protéine synthétisée.

Les ligands glucocorticoïdes synthétiques diffusent librement dans les cellules lorsqu'ils ajoutés dans le milieu de culture ou injectés dans un animal.

Dans ce procédé on utilisera en particulier un ligand synthétique choisi parmi la dexaméthasone, le triamcinolone acétonide, le RU2836, le bimétrazole, le déacylcortivazol et le fluocinolone acetonide.

La présente invention a en outre pour objet une protéine de fusion comprenant un récepteur selon l'invention ou un domaine de liaison du ligand selon l'invention et une protéine dont l'activité est inductible plus fortement par liaison dudit récepteur ou dudit domaine de liaison du ligand (LBD) dudit récepteur avec undit ligand glucocorticoïde synthétique qu'avec un ligand glucocorticoïde naturel.

En particulier, la présente invention a pour objet une protéine de fusion qui comprend une protéine recombinase et plus particulièrement de la famille des intégrases λ et plus particulièrement encore, la protéine Cre du bactériophage P₁.

Dans un mode de réalisation, la protéine de fusion comporte la partie C-terminale de la région charnière D du récepteur nucléaire humain des glucocorticoïdes, intercalée entre la protéine Cre et ledit domaine de liaison du ligand du récepteur modifié selon l'invention.

L'activité recombinase proprement dite de la protéine de fusion est similaire à celle de Cre. Elle permet donc, comme Cre, d'exciser ou d'inverser des séquences d'ADN localisées entre des sites loxP, d'insérer un plasmide contenant un site loxP au niveau d'un site loxP localisé dans le génome, ou d'effectuer une translocation entre deux chromosomes contenant chacun un site loxP.

La présente invention a également pour objet un gène de fusion codant pour la protéine de fusion selon l'invention comprenant un fragment d'ADN codant pour un récepteur nucléaire de glucocorticoïdes modifié ou un domaine de liaison du ligand dudit récepteur selon l'invention, ainsi qu'un fragment d'ADN codant pour une protéine dont on veut réguler l'activité, ladite activité étant inductible par liaison dudit récepteur ou dudit domaine de liaison du ligand avec undit ligand glucocorticoïde synthétique mais pas par liaison avec un ligand glucocorticoïde naturel lorsque ladite protéine se trouve fusionnée audit récepteur ou audit domaine de liaison du ligand dudit récepteur.

Plus particulièrement la présente invention a pour objet un gène de fusion comprenant dans le sens 5' --> 3' :
- un fragment d'ADN codant pour la recombinase Cre du bactériophage P₁ ;
- un fragment d'ADN codant pour tout ou partie de la région charnière D du récepteur nucléaire des glucocorticoïdes, région située entre le domaine DBD et le domaine LBD, et
- le fragment d'ADN codant pour le domaine de liaison du ligand (LBD) modifié, notamment muté, du récepteur nucléaire des glucocorticoïdes selon l'invention.

Et plus particulièrement encore, la présente invention a pour objet, un gène de fusion, caractérisé en ce qu'il a pour séquence , une séquence codant pour la séquence d'acides aminés de l'IDS n° 2 comprenant :
- les acides aminés 1 à 343 qui correspondent à la recombinaison Cre ;
- les acides aminés 346 à 377 qui correspondent à la région C-terminale de la région D du récepteur humain des glucocorticoïdes ;
- les acides aminés 378 à 623 qui correspondent au LBD [GR(I747/T)].

Dans un mode de réalisation particulier, le gène de fusion a substantiellement pour séquence, la séquence Cre-LBD[GR(I747/T)] telle que représentée dans l'IDS n° 2 dont les acides aminés 626 à 667 correspondent à la région F du récepteur humain des oestrogènes.

La présente invention a également pour objet un vecteur d'expression de la protéine de fusion codée par le gène de fusion selon l'invention, dans des cellules hôtes animales notamment de mammifères, caractérisé en ce qu'il comporte le gène de fusion selon l'invention, placé sous le contrôle d'éléments d'expression assurant son expression dans lesdites cellules hôtes. Le vecteur d'expression de la protéine de fusion peut également être un vecteur d'intégration dans le génome des cellules hôtes.

Dans les vecteurs selon l'invention, pour exprimer le récepteur, le LBD ou la protéine de fusion selon l'invention, il faut que les fragments d'ADN codant pour ces molécules soient placées sous le contrôle de séquence de régulation, notamment des séquences promotrices/ enhancer. On peut utiliser en particulier des séquences promotrices/enhancer du virus SV₄₀.

La présente invention a en outre pour objet un procédé de recombinaison, notamment d'excision, insertion, inversion ou translocation conditionnelle au niveau d'un fragment d'ADN contenant un ou deux sites de reconnaissance spécifique d'une protéine recombinase dans des cellules hôtes humaines ou animales, notamment de mammifères, caractérisé en ce qu'il comporte les étapes dans lesquelles :
1) on introduit une protéine de fusion selon l'invention ou un vecteur d'expression de ladite proteine de fusion selon l'invention dans lesdites cellules hôtes dans des conditions permettant l'expression de la protéine de fusion selon l'invention et,
2) on complexe ladite protéine de fusion avec undit ligand glucocorticoïde synthétique, en mettant ledit ligand en présence desdites cellules hôtes.

La présente invention fournit donc un procédé de délétion conditionnelle d'un fragment d'ADN dans lequel on met en oeuvre un procédé d'excision selon l'invention, et dans lequel ledit fragment d'ADN à exciser est intégré entre deux sites de reconnaissance de protéine recombinase orientés en répétition directe. En particulier, ledit fragment d'ADN peut être choisi de telle sorte que l'excision dudit fragment d'ADN a pour effet, l'inactivation dudit gène. L'excision dudit fragment d'ADN peut également permettre la synthèse d'une protéine fonctionnelle, si ledit fragment contient par exemple un codon stop ou un signal de polyadenylation.

Dans le mode préféré de réalisation selon l'invention, lesdits sites de reconnaissance spécifique de la protéine recombinase sont les sites loxP et ladite protéine recombinase est la protéine Cre du bactériophage P₁.

Ledit fragment d'ADN que l'on veut recombiner, notamment exciser et le ou lesdits sites de reconnaissance spécifiques d'une protéine recombinase peuvent être portés par un vecteur plasmidique ou viral, ou intégrés dans le chromosome des cellules hôtes.

L'intégration d'un gène portant des sites loxP dans un génome peut être aléatoire ou ciblée, En particulier, l'intégration des sites de reconnaissance spécifique de la protéine recombinase, notamment du ou des sites loxP pour la recombinase Cre, peut se faire par recombinaison homologue du gène comportant ledit fragment d'ADN à exciser ou inverser (2 sites loxP) ou respectivement inserer ou transloquer (1 site loxP) avec undit gène modifié comportant ledit fragment d'ADN à exciser flanqué en 5' et/ou 3' par le ou lesdits sites de reconnaissance de recombinase en fonction de l'application désirée, notamment les sites loxP.

On a construit une protéine chimérique, Cre-LBD[GR(I747/T)] de l'IDS n° 2, composée de la recombinase Cre fusionnée au LBD de GR (I747/T). La protéine de fusion Cre-LBD[GR(I747/T)] permet de réaliser une recombinaison entre des sites loxP, dans une cellule de mammifère, suite à un traitement par des glucocorticoïdes synthétiques. En l'absence de traitement, ou en présence de concentrations de glucocorticoïdes naturels allant jusqu'à 10-6 M aucune excision n'est observée. Cette chimère est capable d'exciser les séquences d'ADN situées entre des sites loxP intégrés préalablement dans le génome de cellules F9 (embryocarcinome de souris) ou présents sur un plasmide après transfection de ces cellules avec un vecteur exprimant Cre-LBD[GR(I747/T)], suite à un traitement à la Dex à 10-6 M ou 10⁻⁷ M, alors qu'à de telles concentrations, le cortisol n'induit pas d'activité recombinase. Comme on l'a déjà mentionné, le vecteur d'expression de Cre-LBD[GR(I747/T)] peut également être un vecteur d'intégration dans le génome des cellules hôtes.

Ce système permet donc de libérer l'activité recombinase de protéine chimérique à un moment donné et choisi. La protéine de fusion Cre-LBD[GR(I747/T)] peut être exprimée dans des cellules contenant des sites loxP, sans modifier le locus contenant les sites loxP. La recombinaison au niveau des sites loxP a lieu uniquement après traitement aux glucocorticoïdes synthétiques. De plus en exprimant la protéine de fusion Cre-LBD[GR(I747/T)] dans un animal sous le contrôle d'un promoteur à spécificité cellulaire, on peut obtenir une recombinaison entre des sites loxP, spécifiquement dans ces cellules.

La présente invention a également pour objet un vecteur de transfert d'un fragment d'ADN dans des cellules hôtes humaines ou animales, notamment mammifères, caractérisé en ce qu'il comporte undit fragment d'ADN à transférer comprenant des sites de reconnaissance spécifiques de la recombinase, notamment deux sites loxP, orientés à répétitions directe et une cassette d'expression d'un gène de fusion selon l'invention. De façon appropriée les deux sites sont placés à chaque extrémité dudit fragment d'ADN.

De préférence, le gène de fusion est placé sous le contrôle d'éléments d'expression spécifiques des cellules hôtes, en particulier, il comporte la séquence du plasmide pCre-LBD[GR(I747/T)] de l'IDS n° 3.

Dans le procédé d'excision selon l'invention, ledit fragment d'ADN à exciser peut être transféré dans les cellules hôtes avant, en même temps, ou après l'étape d'introduction de la protéine de fusion ou d'un vecteur de transfert.

Le vecteur de transfert selon l'invention peut être, un vecteur plasmidique ou un vecteur viral. Le mode de transfert varie en fonction du type de cellule cible et de l'efficacité recherchée.

Lorsque le matériel est transféré dans les cellules germinales, on utilise de préférence la microinjection du pronucléus mâle d'un ovocyte fécondé. Pour introduire l'ADN dans des cellules embryonnaires pluripotentes (cellules ES), la technique appropriée sera plutôt l'électroporation ou l'utilisation de vecteurs retroviraux

Pour des expériences portant sur les cellules somatiques, on cherche à obtenir une efficacité maximale, d'où l'utilisation préférentielle de vecteurs viraux : rétrovirus et adenovirus, principalement. Ces virus doivent être défectifs, notamment pour une application en santé humaine pour empêcher toute multiplication et tout risque de révertion.

Après intégration ou non de ce type de vecteurs dans le génome, le système Cre-lox permet d'exciser certaines séquences virales, qui pourraient éventuellement présenter un risque quant à la propagation ultérieure du virus. Ceci est très avantageux pour l'utilisation de tels vecteurs en thérapie génique. Des vecteurs contenant une cassette d'expression du gène Cre-LBD[GR(I747/T)] et des sites lox encadrant un gène potentiellement "dangereux" et cette cassette permettent, le cas échéant après intégration dans le génome du virus recombiné, d'éliminer ces éléments. Inversement, on peut également activer un gène que l'on a intégré sous forme inactive. Cette activation peut résulter de la délétion d'un fragment dudit gène par exemple un codon stop ou un signal de polyadénylation encadré de sites loxP.

Lorsque l'on veut transférer du matériel génétique dans une cellule, on utilise en général des constructions comportant le gène à transférer, auquel on a adjoint éventuellement un gène auxiliaire, conférant un avantage sélectif (par exemple, le gène neo, conférant la résistance à l'antibiotique G418).

Les marqueurs de sélection peuvent également être excisés par le procédé d'excision selon l'invention.

En utilisant des techniques classiques, on peut modifier par recombinaison homologue des gènes de mammifères, notamment de souris. Un site loxP peut être introduit dans un gène qu'on veut modifier, ou deux sites loxP peuvent encadrer des séquences que l'on veut modifier. En particulier, le marqueur de sélection utilisé pour permettre d'identifier les évènements de recombinaison homologue peut être gênant, et peut dans un premier temps être éliminé, si nécessaire, s'il est lui même encadré de sites de reconnaissance de recombinase tels que des sites loxP ou FRT, en microinjectant la proteine recombinase correspondante, en transfectant les cellules, notamment des cellules ES, avec un vecteur d'expression pour une protéine recombinase, ou en croisant des souris portant le marqueur de sélection avec des souris exprimant la recombinase désirée, notamment la recombinase Cre. Ceci permet d'obtenir des souris dont la seule modification au niveau du locus modifié est l'insertion de sites de reconnaissance tels que loxP. Ces souris peuvent par la suite être croisées avec des souris exprimant Cre-LBD[GR(I747/T)] et la modification du locus obtenue par traitement avec le ligand tel que la Dex. Ceci permet d'inactiver ou de modifier un gène à un moment déterminé, et donc d'étudier la fonction de ces gènes à différents moments du développement. Ceci est particulièrement intéressant pour l'étude des gènes qui sont indispensables au bon déroulement du développement embryonnaire. En effet, dans certains cas, les techniques classiques de recombinaison homologue entraînent la mort de l'embryon et ne permettent pas d'étudier la fonction du gène à des stades plus tardifs.

Le transfert de gènes dans une cellule donnée est à la base de la thérapie génique. Les vecteurs les plus efficaces à cet égard sont des vecteurs viraux, en particulier rétroviraux ou adénoviraux (39). La présente invention a donc également pour objet un vecteur de transfert selon l'invention comportant ledit fragment d'ADN à transférer comportant deux sites loxP orientés en répétition directe pour une utilisation comme médicament en thérapie génique, lorsque celui-ci est administré en combinaison avec undit ligand glucocorticoïde synthétique tel que la dexaméthasone.

Ce type de vecteur permet de transférer le fragment d'ADN désiré comportant deux sites de reconnaissance spécifique de recombinase dans un gène de sorte que les séquences d'ADN situées entre les deux sites loxP pourront être excisées de façon conditionnelle après administration dudit ligand glucocorticoïde synthétique.

La présente invention fournit en outre des cellules humaines ou animales notamment de mammifères, transfectées par un vecteur d'expression et de transfert d'une protéine de fusion selon l'invention, ou des cellules humaines ou animales notamment de mammifères, dans lesquelles un fragment d'ADN a été transféré à l'aide d'un vecteur de transfert selon l'invention, ou encore des cellules humaines ou animales, notamment de mammifères exprimant de façon constitutive la protéine de fusion selon l'invention.

La présente invention a enfin pour objet supplémentaire, un animal transgénique, notamment une souris comportant un gène fonctionnel de la protéine de fusion selon l'invention, celui-ci étant notamment intégré dans un de ses chromosomes.

Ou encore un animal transgénique comportant un gène fonctionnel de la protéine de fusion selon l'invention, dans lequel un gène d'intérêt est modifié par insertion de site(s) loxP, notamment intégré(s) dans un ou plusieurs chromosomes.

La présente invention a enfin pour objet l'utilisation d'un vecteur d'expression ou de transfert de cellules ou d'un animal selon l'invention comme outil d'analyse ou d'étude du fonctionnement d'un gène donné d'une cellule hôte et une méthode de traitement de cellules ex vivo ou in vitro impliquant la mise en oeuvre d'un procédé d'excision, insertion, inversion ou translocation selon l'invention et l'utilisation d'un vecteur d'expression ou de transfert selon l'invention.

D'autres caractéristiques et avantages de la présente invention apparaîtront à la lumière de la description détaillée qui va suivre.

L'IDS n° 1 représente les séquences nucléotidique et peptidique de de GR(I747/T) avec le DBD correspondant aux aa 421-487 et le LBD correspondant aux aa 532-777;

L'IDS n° 2 représente les séquences nucléotidique et peptidique de Cre-LBD[GR(I747/T)]

Les nucléotides 1-1029 codent pour la recombinase Cre (aa 1-343).

Les nucléotides 1036-1131 codent pour la région C-terminale de la région D du récepteur humain des glucocorticoïdes [aa 500-531 de GR(I747/T) ; aa 346-377 de Cre-LBD[GR(I747/T)].

Les nucléotides 1132-1869 codent pour le LBD de GR(I747/T) [aa 532-777 de GR(I747/T) ; aa 378-623 de Cre-LBD[GR(I747/T)].

Les nucléotides 1876-2001 codent pour la région F du récepteur humain des oestrogènes [aa 554-595 de HEG0 ; aa 626-667 de Cre-LBD[GR(I747/T)].

Les nucléotides 1777-1779 correspondent à la mutation qui remplace l'isoleucine par une thréonine.

L'IDS n° 3 représente la séquence nucléotidique du plasmide pCre-LBD[GR(I747/T)]

Les IDS n° 4 à 17 représentent les séquences nucléotidiques des différents oligonucléotides utilisés selon l'invention.

La Figure 1 est une représentation schématique d'activités recombinases de Cre.

La Figure 2 représente l'activité transcriptionnelle de récepteur GR mutant et de type sauvage en réponse à la dexaméthasone et la capacité de fixation de la dexaméthasone aux deux récepteurs.

La Figure 3 représente une analyse de Scatchard de la liaison de [³H]-dexaméthasone aux récepteurs de type sauvage et mutant 1747/T.

La Figure 4 représente la compétition par RU 486 des activités de liaison de Dex et de transactivation du récepteur de type sauvage (TS) et GR(I747/T).

La Figure 5 est une représentation schématique des protéines Cre, hER, Cre-ER, hGR et Cre-LBD[GR(I747/T)].

La Figure 6 est une représentation schématique du plasmide pCre-LBD[GR(I747/T)].

La Figure 7 représente la séquence nucléotidique de pCre-LBD[GR(I747/T)] et la séquence peptidique de Cre-LBD[GR(I747/T)].

La Figure 8 représente la stratégie PCR utilisée pour détecter les allèles sauvages, mutés et recombinés dans le test d'activité recombinase de Cre-LBD[GR(I747/T)].

La Figure 9 représente l'analyse par PCR de l'excision des séquences situées entre les sites loxP après transfection transitoire de pCre-LBD[GR(I747/T)] dans les cellules murines RXRα^{+/-(LNL)} sans ajout de ligand, ou traitées au cortisol (10⁻⁶ M) ou à la dexaméthasone (10⁻⁶ M).

La Figure 10 représente l'analyse par PCR de l'activité recombinase de Cre-GR(I747/T) dans les cellules RXRα^{+/-(LNL)} : Cre-GR(I747/T) non traitées ou traitées avec du cortisol ou de la dexaméthasone à 10⁻⁶M.

La Figure 11 récapitule l'activité recombinase de Cre-LBD[GR(I747/T)] dans la lignée RXRα^{+/-(LNL)} exprimant la recombinase de façon constitutive, en présence de concentrations croissantes de différents ligands.

La Figure 12 représente l'analyse par PCR de l'activité recombinase de Cre-LBD[GR(I747/T)] induite par des ligands synthétiques malgré la présence de ligands naturels.

La Figure 13 représente le test de l'activité recombinase de Cre-LBD[GR(I747/T)] dans les cellules humaines HeLa.

La Figure 14 est une représentation schématique du trangène Cre-LBD[GR(I747/T)]. La partie (a) représente le fragment d'ADN contenant la séquence codante du gène Cre-LBD[GR(I747/T)] utilisé pour établir des souris transgéniques et la partie (b) représente la structure génomique de l'allèle RXRα de type sauvage, de l'allèle cible RXRα^{ΔAF2(LNL)} et de l'allèle excisé RXRα^{Δ(AF2(L)} ainsi que la stratégie PCR pour l'analyse de l'excision du marqueur.

La Figure 15 représente l'analyse par PCR de l'activité recombinase de Cre-LBD[GR(I747/T)] chez la souris traitée à la dexaméthasone.

### EXEMPLE 1 : Remplacement de l'isoleucine 747 par la thréonine dans le récepteur nucléaire humain des glucocorticoïdes.

On a obtenu un mutant GR humain comportant un seul remplacement d'acide aminé, celui du résidu isoleucine 747 par un résidu thréonine, localisé entre les hélices H11 et H12, dans la partie C-terminale du domaine de liaison du ligand. Cette mutation est apparue spontanément dans les vecteurs dérivés de HG1 (40). Ce mutant diffère des autres mutants de LBD de GR décrits précédemment (22, 38), dont la fonction d'activation altérée est en étroite corrélation avec une modification de leurs propriétés de liaison du ligand in vitro.

### 1. Mutation dans le GR humain

Le vecteur d'expression du GR humain de type sauvage (pSG5HG0) a été obtenu en clonant le fragment BamHI d'environ 2.9 kb (contenant le cadre de lecture ouvert du hGR de type sauvage) isolé du vecteur HG0 précédemment décrit (40), dans le site BamHI du vecteur d'expression pSG5 (41).

Toutes les constructions d'ADN ont été effectuées au moyen de techniques classiques (42). Le vecteur d'expression GR (I747/T)hGR codant pour un mutant portant la mutation I 747->T, a été obtenu à partir du fragment de digestion PstI-PflMI du vecteur d'expression HG1 (40) muté qui était ligaturé dans les sites PstI et PflMI correspondants de pSG5HG0.

En plus de la mutation du résidu isoleucine 747 en un résidu thréonine présente dans HG1, ce mutant contient également une mutation du site EcoRI qui n'entraîne pas d'altération de la séquence peptidique.

La présence de la mutation a été confirmée par séquencage (protocole Sequenase 2.0, U.S. Biochemical Corp., Cleveland, HO).

### 2. Résultats

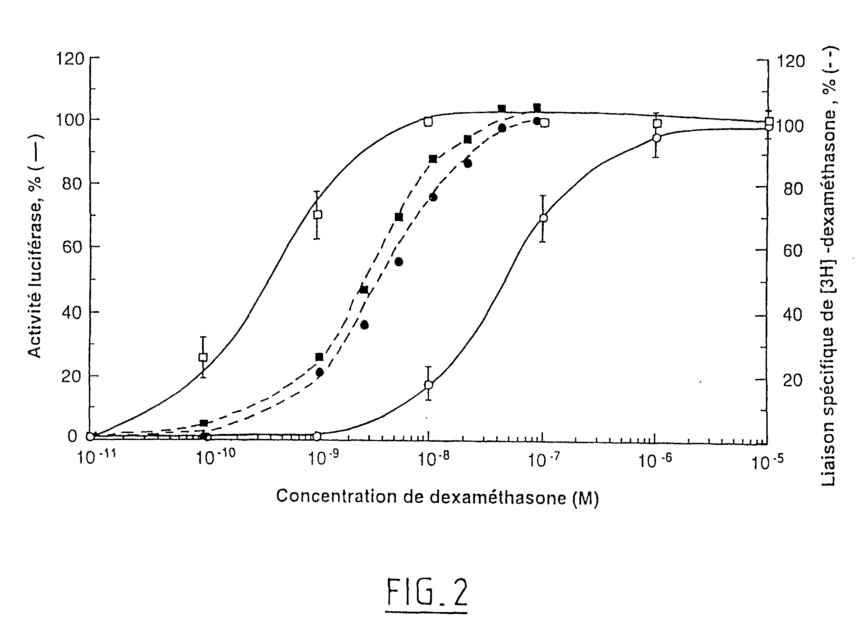

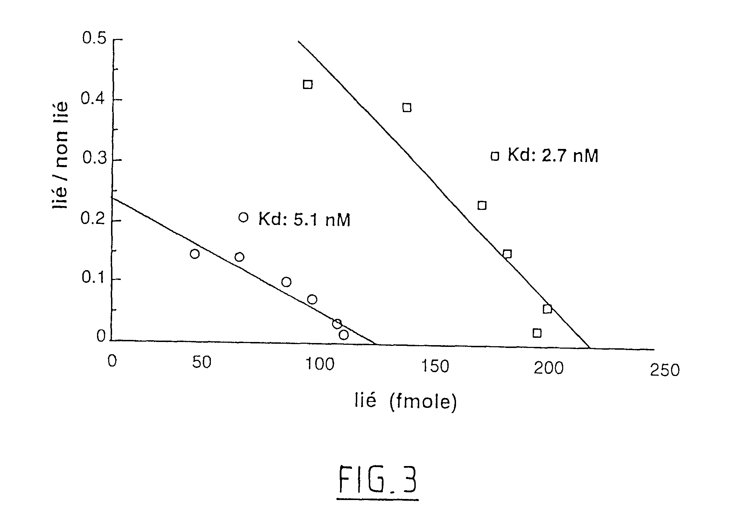

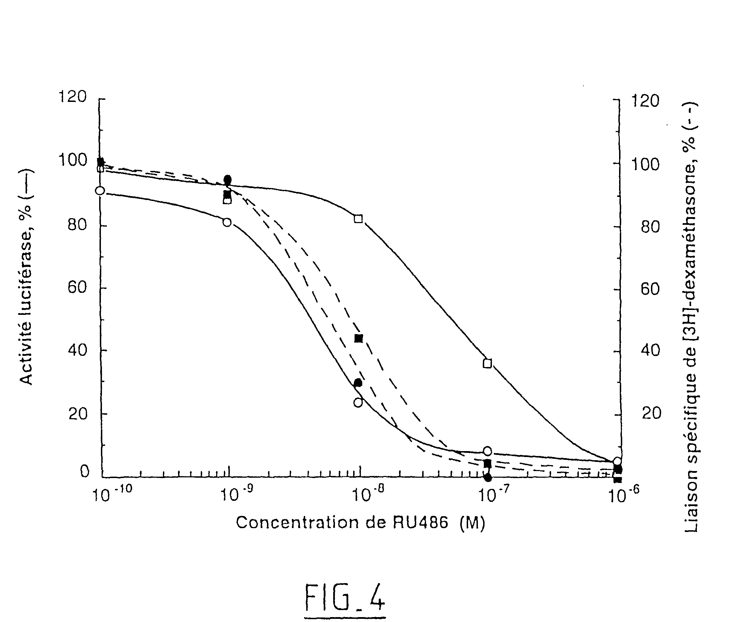

2.1. Le remplacement de l'isoleucine 747 par la thréonine conduit à un décalage de la courbe de réponse en fonction de la dose de dexaméthasone pour la transactivation, sans affecter sensiblement l'affinité de liaison du ligand.
On a comparé les caractéristiques transcriptionnelles du hGR de type sauvage (TS) et d'un mutant dans lequel l'isoleucine 747 a été remplacée par la thréonine [désigné ci-après par GR(I747/T)]. Les récepteurs ont été exprimés transitoirement dans des cellules CV-1 exemptes de GR, conjointement avec un gène reporter de luciférase sous le contrôle du promoteur de l'aminotransférase de la tyrosine (pTAT-tk-Luc). La Figure 2 montre les courbes correspondantes de réponse en fonction de la dose de ligand utilisée, permettant de déterminer les valeurs de la CE50 (la concentration de ligand donnant 50 % de la réponse transcriptionnelle maximale). La courbe de réponse en fonction de la dose pour hGR TS présente une réponse maximale à 10-8 M de Dex et une réponse égale à la moitié de la réponse maximale à 4x10⁻¹⁰ M, alors que la courbe de réponse transcriptionnelle du mutant GR(I747/T) est nettement décalée vers les plus fortes concentrations de ligand, entraînant des valeurs 100 fois plus élevées de la CE₅₀ pour Dex (4x10-8 M) (voir tableau I ci-après). La stimulation maximale médiée par GR(I747/T) a été atteinte à environ 10-6 M Dex. Toutefois, il est important de noter que le récepteur GR(I747/T) est capable d'activer la transcription du pTAT-tk-Luc au même degré que le GR TS. En conséquence, le remplacement du résidu isoleucine par la thréonine à la position 747 ne diminue pas l'aptitude du GR à activer la transcription mais a modifié spécifiquement la sensibilité au ligand de la réponse.
L'effet de cette mutation est indépendant de la région A/B N-terminale et de sa fonction de transactivation associée AF-1. Les mutants de délétion dépourvus de AF-1 mais contenant la mutation I747/T[ΔA/B-GR(I747/T)] nécessitent 3x10⁻⁸ M de Dex pour une stimulation représentant la moitié de la stimulation maximale, tandis que seulement 6x10⁻¹⁰ M sont suffisants pour le ΔA/B-GR dérivé de TS (données non représentées; voir également le tableau II pour les chimères GAL correspondantes). Ainsi, la mutation I747/T exerce un effet similaire sur le GR complet et le GR à délétion N-terminale. On peut en conclure que le décalage observé dans la réponse en fonction de la dose de ligand n'est pas la conséquence d'une interaction modifiée entre AF-1 et AF-2.
Pour déterminer l'effet de la mutation I747/T sur la liaison dU ligand, on a mesuré la liaison de Dex dans un cytosol de cellules Cos-7 exprimant transitoirement le hGR mutant et le hGR de type sauvage. Les valeurs de K_{d} obtenues à partir d'une analyse de Scatchard sont très semblables, puisque le mutant 1747/T ne manifeste qu'une affinité pour la Dex deux fois plus faible que le GR TS (Figure 3). Ainsi, l'ordre de grandeur du décalage des courbes dose-réponse vers les fortes concentrations de hGR (I747/T), par rapport au hGR TS (environ 100 fois) est peu susceptible de résulter d'une diminution d'affinité pour le ligand du hGR muté (facteur 2 seulement ; comparer les Figures 2 et 3).
2.2. hGR(I747/T) ne répond pas aux ligands naturels
Pour caractériser davantage les caractéristiques de liaison du mutant I747/T, on en a comparé l'activation par divers agonistes synthétiques connus pour le GR TS (triamcinolone acétonide, RU28362, bimédrazol), un agoniste partiel (fluocinolone acétonide) et des glucocorticoïdes naturels (cortisol, cortico-stérone). Avec le récepteur mutant, le triamcinolone acétonide et le RU28362 donnent la même transactivation qu'avec le récepteur de type sauvage, tandis que le bimédrazol et la fluocinolone acétonide ont manifesté une plus forte activité (tableau I). Pour tous ces analogues, le mutant 1747/T manifeste un décalage de la courbe de réponse en fonction de la dose, en direction des plus fortes concentrations de ligand, bien que ce décalage soit moins prononcé pour des ligands à forte affinité (tableau I).
Les ligands naturels, tels que le cortisol ou la corticostérone ou l'aldostérone, le ligand naturel du récepteur de minéralocorticoïdes, se lient au GR avec une plus faible affinité que Dex. Avec le GR de type sauvage, ces ligands sont aussi efficaces que la Dex pour stimuler le reporter de la luciférase (tableau I). Toutefois, en présence de ces ligands naturels, et en contraste notable avec les effets observés avec Dex, la transactivation par le mutant I747/T est fortement diminuée (cortisol) ou supprimée (corticostérone, aldostérone).
2.3. Activités agonistes et antagonistes similaires de RU486 avec hGR de type sauvage et hGR mutant
RU486 est un puissant inhibiteur de l'expression des gènes dépendant des glucocorticoïdes. En présence de cet antagoniste, la fonction AF-2 de GR est bloquée, mais on a pu observer une certaine activité agoniste dépendant de AF-1 [(43) et références qui y sont contenues]. Une mutation ou une délétion de la partie C-terminale du LBD peuvent altérer la réponse à des ligands agonistes/antagonistes, bien que les rôles de AF-1 et de AF-2 n'aient pas été clairement définis dans les études rapportées (44-46).
Pour tester l'affinité de liaison relative de RU486 pour le mutant 1747/T et le type sauvage, on a examiné l'aptitude de RU486 à entrer en compétition avec la Dex pour la liaison aux récepteurs. Des cellules transfectées avec hGR mutant ou TS ont été traitées par 10 nM de [3H]-Dex, une concentration suffisante pour engendrer essentiellement des récepteurs mutants et TS complexés avec la Dex, conformément aux essais de liaison de ligand in vitro (voir plus haut), et par des concentrations croissantes de RU486 (Figure 4) . On a obtenu des valeurs de la CI₅₀ (la concentration d'antagoniste bloquant 50 % de la transactivation maximale) très similaires pour les deux récepteurs (10⁻⁸M pour le hGR TS et 6x10⁻⁹M pour le hGR mutant). Ces résultats sont en accord avec les essais de liaison à Dex et montrent que les affinités de liaison à Dex et RU486 ne sont pas modifiées par la mutation I747/T.
2.4. Le décalage de l'activité transcriptionnelle du récepteur mutant est indépendant du promoteur
On a obtenu les résultats décrits ci-dessus dans des cellules transfectées, en utilisant un gène rapporteur contenant un fragment promoteur du gène TAT qui héberge deux éléments de réponse aux glucocorticoïdes (GRE). Pour distinguer entre une modification des propriétés intrinsèques de transactivation du récepteur et une modification de l'activité coopératrice résultant de la liaison de plusieurs molécules de récepteur, on a comparé les activités transcriptionnelles du récepteur mutant GR(I747/T) et du récepteur de type sauvage, en utilisant un promoteur synthétique contenant un seul GRE, palindromique et parfait. Les courbes correspondantes de réponse en fonction de la dose présentent les mêmes profils que celles obtenues avec le promoteur TAT. La réponse correspondant à la moitié de la réponse maximale est obtenue à 10⁻¹⁰ M pour le GR TS et à 2x10⁻⁸ M pour GR(I747/T) (tableau II). Cela indique que le décalage observé avec le récepteur mutant reflète les propriétés de transactivation intrinsèques du mutant GR(I747/T) et ne sont pas le résultat d'une altération de la coopération entre les récepteurs.
Le remplacement du domaine de liaison d'ADN de GR par les 147 premiers acides aminés du transactivateur de Gal4 de levure donne une protéine activatrice hybride ayant une nouvelle spécificité de promoteur. Les hybrides Gal4-GR consistent en le domaine de liaison d'ADN (DBD) de Gal4 (1-147) fusionné avec le LBD de hGR TS ou mutant. Leurs aptitudes à activer la transcription ont été testées dans des essais de transfection transitoires dans des cellules HeLa. On a constaté le même décalage qu'avec le mutant GR(I747/T) complet (tableau II). Ces résultats montrent que le décalage de la réponse transcriptionnelle en fonction de la dose du ligand est indépendant de promoteur et d'élément de réponse (RE). En outre, cet effet est apparemment non spécifique des souches de cellules utilisées, comme on l'a constaté à la fois dans des cellules humaines HeLa et des cellules simiennes Cos. Pour exclure davantage une implication possible de facteurs se liant au promoteur tk ou β-globine du gène rapporteur (47), on a utilisé un gène rapporteur contenant un élément de réponse Gal4 précédant un promoteur minimal. Bien que la transactivation par la protéine chimérique Gal4 ait été diminuée avec ce gène rapporteur minimal, on a encore pu observer un décalage de la courbe de réponse vers les doses croissantes pour la protéine chimérique mutante Gal4-GR(1747/T) (tableau II).
2.5. Activité transcriptionnelle de récepteurs GR mutant et de type sauvage en réponse à la dexaméthasone (Figure 2).
Des cellules CV-1 ont été transitoirement transfectées avec 0,5 µg de vecteurs d'expression de GR de type sauvage (□) ou de GR(I747T) (o), 2,5 µg de pTAT-tk-Luc en tant que plasmide raporteur et 0,5 µg de pCMV-βGal en tant que témoin interne. Après la transfection, on a mis les cellules à incuber pendant 24 heures avec des concentrations croissantes de Dex, et on les a ensuite soumises à un essai des activités β-galactosidase et luciférase, comme décrit dans "Matériels et Méthodes". L'activité luciférase a été normalisée avec l'activité β-galactosidase, et la valeur obtenue en absence de ligand a été soustraite pour chaque point. Les résultats ont été exprimés en pourcentage de l'activité luciférase maximale obtenue pour chaque récepteur. Chaque point est la moyenne ± écart-type de la moyenne, de quatre déterminations séparées effectuées en double. Les analyses de liaison à saturation ont été effectuées avec des cytosols provenant de cellules Cos-7 transfectées avec 40 µg de vecteur d'expression de hGR de type sauvage (□) ou de hGR(I747/T) (•), avec 20 µg de pCMV-βGal mis à incuber pendant une nuit à 4°C en présence de concentrations croissantes de [³H]-dexaméthasone ± un excès de 100x de Dex radio-inerte. Le stéroïde [³H] fixé a été mesuré après traitement au charbon enrobé de dextran. Les résultats représentés sont ceux d'un seul essai effectué en double et représentatif de trois essais séparés. Les courbes de saturation de la liaison de [3H]-dexaméthasone sont représentées en pourcentage de la capacité de liaison maximale obtenue pour chaque récepteur. La capacité de liaison du mutant GR(I747/T) est diminuée pour atteindre environ 40-60 % de celle du hGR de type sauvage (623 ± 65 fmoles/mg de protéine, par rapport à 1 340 ± 285 fmoles/mg de protéine).
2.6. Analyse de Scatchard de la liaison de [³H] -dexaméthasone aux récepteurs de type sauvage et mutant 1747/T (Figure 3).
On a appliqué l'analyse de Scatchard à la liaison effectuée comme décrit sur la Figure 2. La constante de dissociation apparente du mutant GR(I747/T) (o) n'est que deux fois plus élevée que celle du hGR de type sauvage (□) (K_{d} = 5,1 ± 1,9 par rapport à 2,7 ± 1,3 nM).
2.7. Tableau I
Activités transcriptionnelles et CE₅₀ de divers stéroïdes avec hGR de type sauvage et hGR(I747/T)

| | Activité luciférase % | | | CE50 | |
|---|---|---|---|---|---|
| Ligand | Type sauvage | hGR (I747/T) | Type sauvage | hGR (I747/T) | %CE₅₀ |
| Dexaméthasone | 100 | 100 | 4x10⁻¹⁰M | 4x10⁻⁸M | 100 |
| TA | 98 | 102 | 5x10⁻¹⁰M | 2x10⁻⁸M | 40 |
| RU28362 | 90 | 88 | 3x10⁻¹⁰M | 7x10⁻⁹M | 23 |
| Fluocinolone | 75 | 100 | 1,5x10⁻¹⁰M | 3x10⁻⁹M | 20 |
| Bimédrazol | 100 | 180 | 10⁻¹⁰M | 2x10⁻⁹M | 20 |
| Cortisol | 110 | 15 | 7x10⁻⁹M | nm | |
| Corticostérone | 100 | 0 | 7x10⁻⁸M | nm | |
| Aldostérone | 90 | 0 | 5x10⁻⁸M | nm | |

Les co-transfections ont été effectuées et les courbes de réponse en fonction de la dose ont été construites comme décrit sur la Figure 2. On a testé à six concentrations croissantes, en double, le triamcinolone acétonide (TA), RU28362, le fluocinolone acétonide (fluocinolone), le cortisol, la corticostérone et l'aldostérone. Les essais de l'activité luciférase et de la β-galactosidase ont été effectués comme décrit dans "Matériels et Méthodes". Les moyennes de trois essais indépendants sont données dans ce tableau. nm = non mesurable.
2.8. Compétition des activités de liaison de Dex et de transactivation par RU486 (Figure 4).
Des cellules Cos-7 transfectées avec le hGR de type sauvage (□) ou mutant I747T (•) ont été mises à incuber pendant 1 heure à 37°C avec de la [3H]-dexaméthasone 10 nM seule ou en présence de la concentration indiquée de RU486 non marqué, comme décrit dans "Matériels et Méthodes". Les données sont exprimées en pourcentage de la liaison spécifique observée sur le témoin non soumis à la compétition. Ces données représentent la moyenne de déterminations effectuées en double pour chaque concentration d'antagoniste.
L'activité antagoniste a été déterminée sur des cellules CV-1 transfectées comme décrit sur la Figure 4 et traitées simultanément par Dex 10⁻⁷M et la concentration indiquée de RU486, 24 heures avant la récolte pour l'essai des activités luciférase et β-galactosidase. 100 % d'activité sont définis pour chaque récepteur comme l'activité luciférase des cellules traitées par la Dex seule. Chaque point représente la moyenne d'au moins deux essais indépendants effectués en double. (□): hGR de type sauvage; (o): mutant I747T.
2.9

**Tableau II**

| CE₅₀ obtenue avec divers gènes reporter | | |
|---|---|---|
| Récepteur | Gène rapporteur | CE₅₀ |
| GR de type sauvage | pGRE-tk-Luc | 10⁻¹⁰M |
| GR(I747/T) | " | 2x10⁻⁸ M |
| Gal4-GR | p(17M)₅-βGlob-Luc | 1,7x10⁻⁹M |
| Gal4-GR_{(I747/T)} | " | 1,5x10⁻⁷M |
| Gal4-GR | p(17M)₅-tata-Luc | 2x10⁻⁹M |
| Gal4-GR_{(I747/T)} | " | 2x10⁻⁷M |

Des cellules HeLa (pour les chimères Gal4) ou des cellules CV-1 ont été cotransfectées transitoirement avec divers vecteurs d'expression et gènes reporter. L'efficacité de la transfection a été standardisée en cotransfectant par 0,5 µg de vecteur pCMV-βgal. Les cellules ont été traitées pendant 24 heures par des concentrations croissantes de dexaméthasone. Les essais de la luciférase et de la β-galactosidase ont été effectués comme décrit dans "Matériels et Méthodes". L'activité luciférase est exprimée en pourcentage de l'activité maximale obtenue pour chaque récepteur et chaque gène reporter. Les valeurs de la CE₅₀ ont été déterminés graphiquement.
2.10. Récapitulatif
La mutation de l'isoleucine 747 du récepteur humain des glucocorticoïdes en thréonine résulte en une très forte diminution, voire une abolition de l'activité de transactivation du récepteur, en présence de glucocorticoïdes naturels, alors qu'à des concentrations similaires, des glucocorticoïdes synthétiques stimulent de façon efficace l'activité de transactivation de ce récepteur muté.

Il est important de souligner que hGR(I747/T) répond totalement à une forte concentration de Dex, ce qui indique qu'il peut en principe transactiver aussi efficacement que sa contrepartie de type sauvage. En conséquence, la fonction d'activation transcriptionnelle de AF-2 elle-même n'est apparemment pas altérée par la mutation, comme c'est le cas pour des récepteurs comportant des mutations dans la partie centrale de AF-2 (voir l'introduction pour les références).

### 3. Matériels et méthodes

### 3.1. Cultures cellulaires et transfection

Les cellules CV-1 et Cos-7 ont été cultivées dans du milieu d'Eagle modifié par Dulbeco (DMEM) contenant 10 % (v/v) de sérum de veau foetal (SVF) (Gibco, Grand Island, NY), dans une atmosphère humidifiée contenant 5% de CO₂. Les cellules HeLa ont été cultivées dans les mêmes conditions. Les transfections transitoires ont été effectuées dans des multi-plaques à 6 puits, selon le mode opératoire au phosphate de calcium déjà décrit (42). Un plasmide recombinant de référence, pCMV-βGal, exprimant la β-galactosidase bactérienne, a été transfecté conjointement avec le vecteur d'expression du récepteur et le gène reporter correspondant, afin de corriger d'éventuelles variations de l'efficacité de la transfection. Le précipité a été éliminé par lavage au bout de 24 heures, et les cellules ont été maintenues dans du DMEM sans SVF mais contenant de l'hormone. Au bout d'encore 24 heures, les cellules ont été rincées et lysées pendant 15 minutes avec 0,3 ml de tampon de lyse [Tris.phosphate 25mM, pH 7,8, MgCl₂ 8 mM, 1% de Triton X100, 10 % de glycérol (48)]. L'activité luciférase correpondante a été déterminée sur une fraction aliquote (0,1 ml), par évaluation du pic de luminescence (pour un temps d'intégration de 15 secondes), après injection de 0,1 ml de luciférine 1 mM dans un luminomètre LKB. La β-galactosidase a été déterminée par la méthode modifiée par Bocquel et al. (49), pour le contrôle de l'efficacité de chaque transfection.

### 3.2. Plasmides utilisés

Les GR à délétion N-terminale, ΔA/B-GR ou ΔA/B-GR5I747/T), qui exprimaient seulement les acides aminés 368 à 777, ont été obtenus à partir du fragment de digestion P*st*I-P*fl*MI du vecteur d'expression HG0 ou pGR(I747/T), respectivement, ligaturé dans les sites correspondants de HG8 (49). Le plasmide pGAL-GR (don de T. Lerouge) était constitué du domaine de liaison à l'ADN de la protéine Gal4 de levure (aa1-147) et du domaine de liaison à une hormone de GR (a500-777); pGal-GR(1747/T) a été construit par insertion de la mutation dans pGal-GR.

Le gène reporter pTAT-tk-Luc a été fourni par D. Gagne et a été construit comme suit: on a d'abord engendré un plasmide intermédiaire pTAT, par insertion du segment EcoRI-BamHI de 966 pb, provenant de pKT531 [fourni gracieusement par T. Grande (50)] dans les sites correspondants du vecteur pUC19 (Pharmacia). Le fragment contenant la séquence codant pour la luciférase de luciole et le promoteur minimal thymidine kinase (tk) provenant du virus Herpes simplex a été isolé sous forme d'un fragment BamHI à partir de pvit-tk-Luc (51) et inséré, en aval du promoteur TAT, dans le site correspondant de pTAT. Le plasmide reporter pGRE-tk-Luc a été construit par ligature du fragment de digestion HindIII-BglII de pGRE-tk-CAT (52), qui contient un GRE synthétique (AGAACAcagTGTTCT) en amont du promoteur minimal tk, dans les sites HindIII-BglII de pLuc (53). Le gène reporteur p(17M)₅-βGlob-Luc a été décrit précédemment (54). Le plasmide reporter p(17M)₅-tata-Luc a été construit à partir du plasmide p(17M)₅-tata-CAT (55) par digestion par XhoI-BamHI et isolement du fragment contenant (17M₅)-tata. Ce fragment a été ensuite inséré dans le vecteur pGL₂ (Promega) digéré par les mêmes enzymes de restriction.

Le vecteur d'expression de la β-galactosidase pCMV-βGal contient la séquence codant pour la β-galactosidase, sous contrôle du puissant promoteur constitutif de cytomégalovirus (CMV).

### 3.3. Essai de liaison à une hormone

Des cellules Cos-7 transfectées avec pCMV-βGal et hGR de type sauvage ou mutant I747/T ont été récoltées 48 heures après la transfection, dans une solution salée tamponnée au phosphate (STP), par grattage à l'aide d'une spatule en caoutchouc. Les cellules ont été lavées deux fois dans de la STP glacée. Toutes les étapes de la préparation de cytosols ont été effectuées à 4°C. On a remis les cellules en suspension dans 3 volumes de tampon de liaison [Na-HEPES 20 mM, pH 7,3, molybdate de sodium 20 mM et EDTA 5 mM (22)] et avec des inhibiteurs de protéase [leucopeptine (5 µg/ml), aprotinine (5 µg/ml), PMSF (40 µg/ml) et pepstatine A (5 µg/ml)], et on les a placées pendant 15 minutes sur de la glace. Les cellules ont ensuite été lysées par 30-40 coups de piston dans un homogénéisateur en verre Dounce, et l'homogénat résultant a été ensuite centrifugé à 110 000 g pendant 30 minutes. Le surnageant, dénommé cytosol, a été utilisé immédiatement pour l'essai de liaison à une hormone. Les concentrations de protéine ont été déterminées par le dosage de protéine Bio-Rad (Bio-Rad Laboratories, Hercules, CA, USA). Pour l'essai de liaison à une hormone, on a mis à incuber pendant une nuit à 4°C un cytosol, contenant 1-3 mg de protéine/ml, avec de la [3H]-Dex (49 CI/mmole; Radiochemical Center, Amersham, Angleterre) ou du cortisol (67 Ci/mmole; Radiochemical Center, Amersham, Angleterre) sans (liaison totale) ou avec (liaison non spécifique) un excès de 100 fois de composé radio-inerte (10⁻⁵ M, Sigma Chemical Co., St. Louis, Mo, USA). On a ensuite traité les échantillons par 1 volume de charbon-dextran (5% de charbon, 0,5% de dextran dans la solution de liaison) pendant 15 minutes dans de la glace, sous agitation constante, puis on les a soumis à une centrifugation pendant 10 minutes à 12 000 g. Le ligand [³H] fixé a été mesuré dans une partie aliquote du surnageant, par comptage de scintillation. La constante de dissociation à l'équilibre (Kd) du récepteur pour la dexaméthasone ou le cortisol a été déterminée par analyse de Scatchard.

Pour l'analyse de liaison compétitive, on a mis les cytosols à incuber avec 10 nM de [3H]-Dex et diverses concentrations de stéroïdes compétitifs non radioactifs, dans les mêmes conditions que précédemment. Après traitement au charbon-dextran et comptage de scintillation, la liaison spécifique a été exprimée en pourcentage du témoin non soumis à une compétition, et portée en fonction de la concentration du stéroïde en compétition. Des essais de liaison compétitive dans des cellules entières ont été effectués sur 106 cellules mises à incuber pendant 1 heure à 37°C avec 10 nM de [3H]-Dex et diverses concentrations de stéroïdes en compétition. Après sonication dans 1 ml de tampon KCl (EDTA 1,5 mM, Tris.HCl 20 mM, pH 7,4, KCl 0,5 M), on a éliminé le stéroïde non lié par addition de deux volumes de charbon enrobé de dextran, dans du tampon KCl à la gélatine (0,05 % de dexiran, 0,5% de charbon, 0,1% de gélatine). On a mis le mélange à incuber pendant 30 minutes à 4°C et on l'a centrifugé à 3 000 g pendant 5 minutes. La radioactivité a été déterminée par comptage de scintillation en liquide et on a porté sur une courbe comme précédemment la liaison spécifique.

### EXEMPLE 2 : Recombinase Cre chimérique dont l'activité est inductible par des glucocorticoïdes synthétiques, mais pas par des glucocorticoïdes naturels

### 1. Matériel et méthodes

### 1.1. Construction du vecteur pCre-LBD[GR(I747/T)]

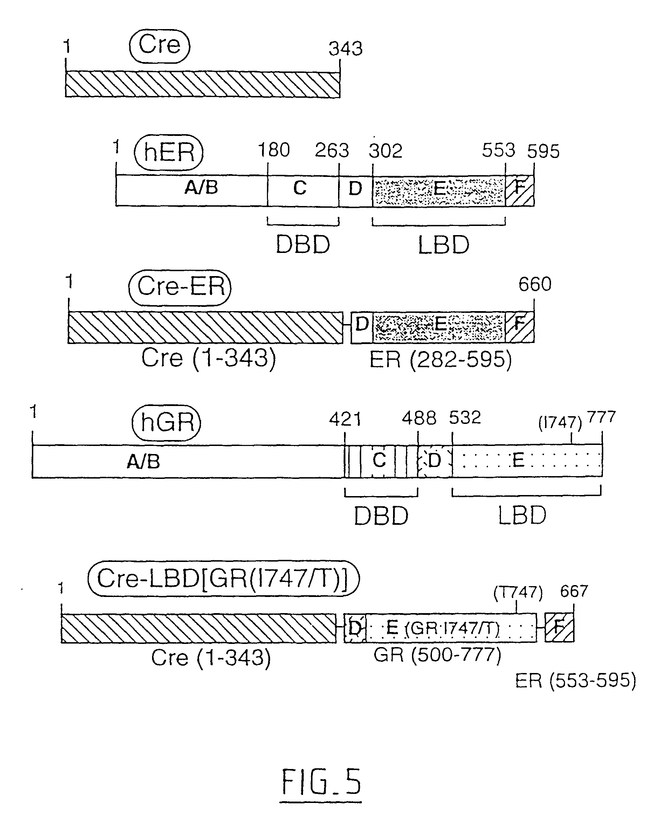

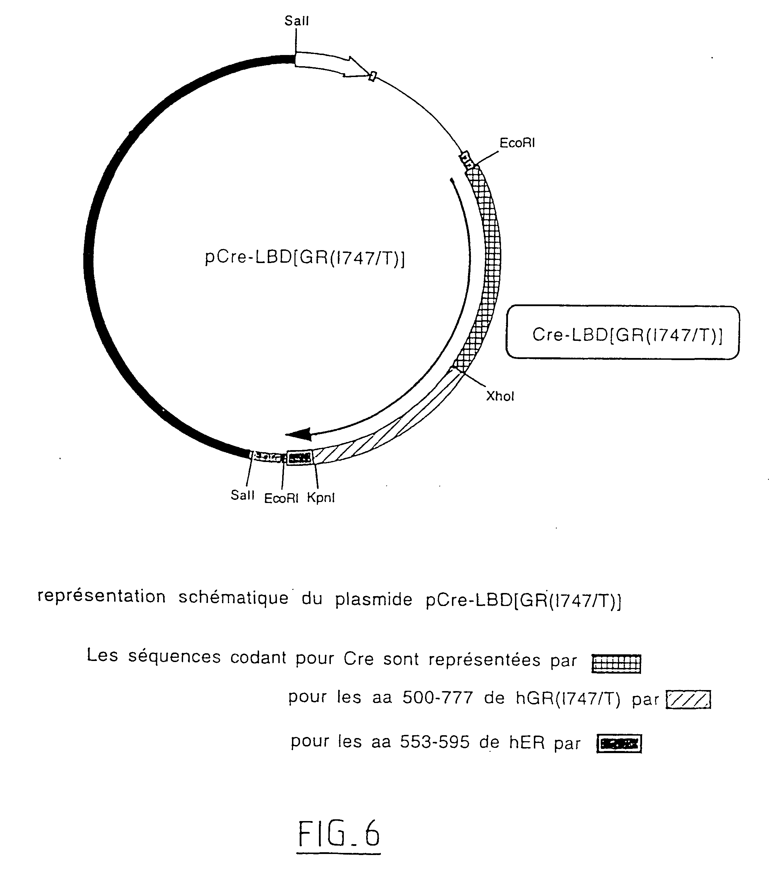

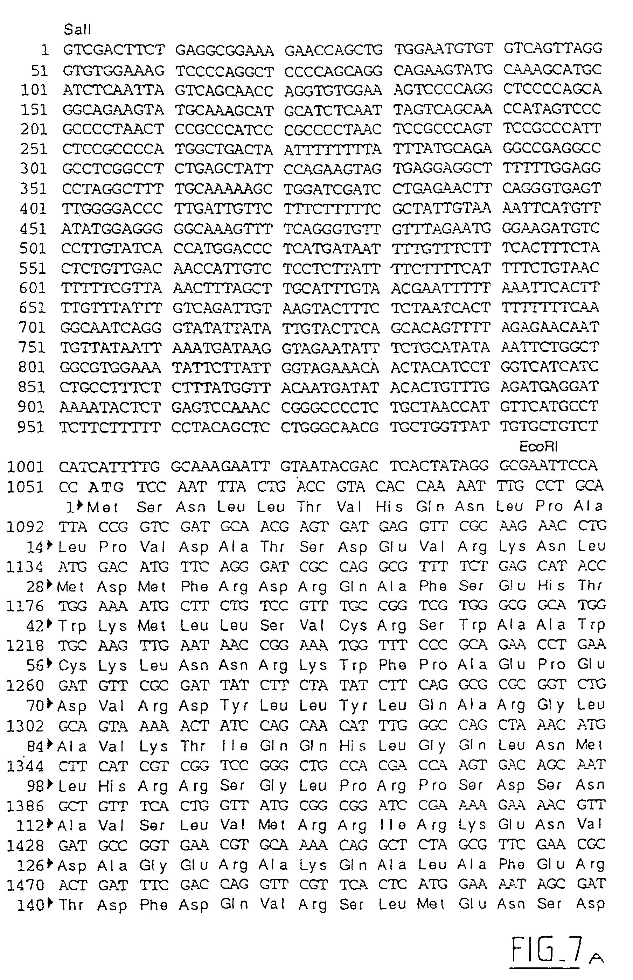

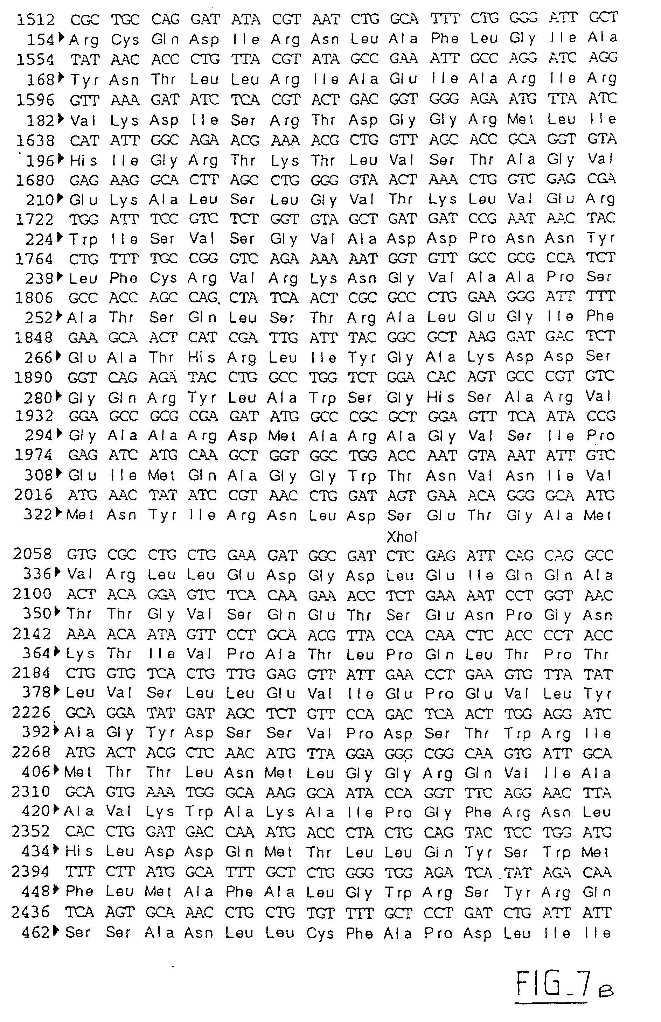

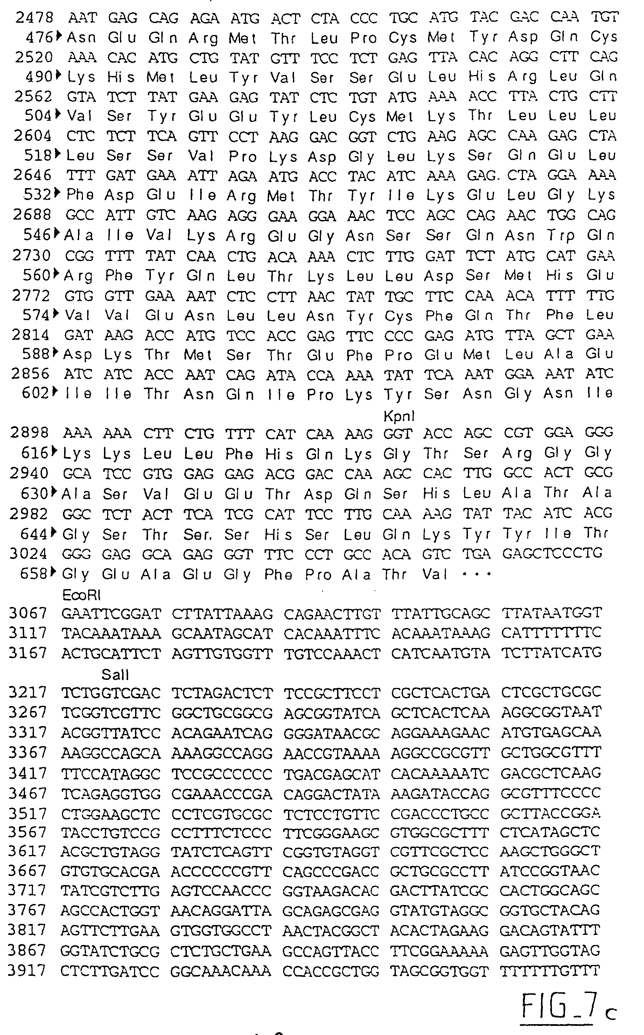

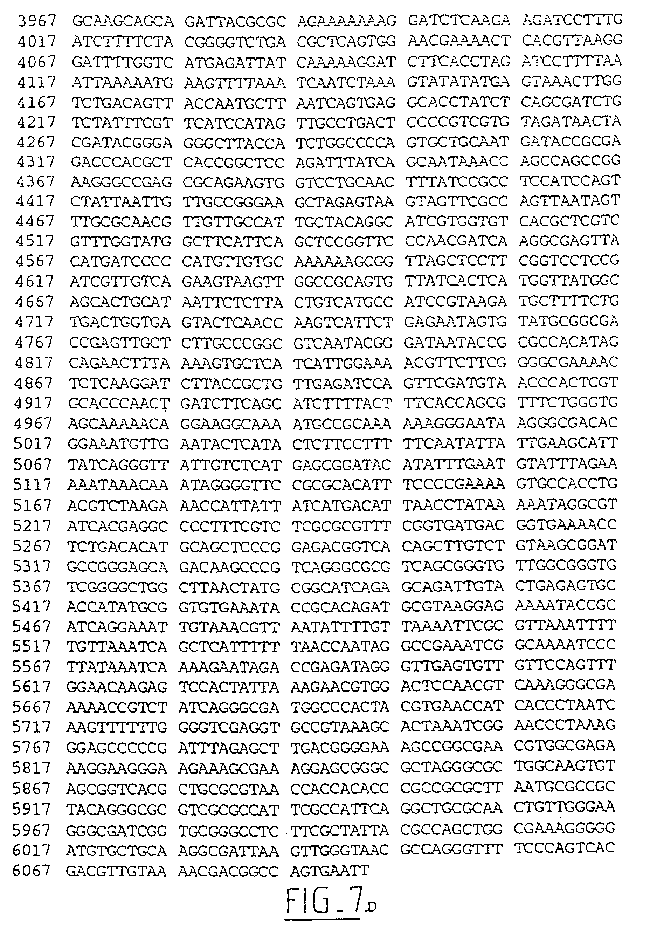

Un fragment d'ADN ClaI-XbaI d'environ 1300 paires de base (pb), contenant la séquence codant pour le domaine de liaison du ligand du récepteur humain des glucocorticoïdes a été isolé du plasmide HG1 (voir Exemple 1 (40)) et ses extrémités ont été remplies par la T4 polymérase. Ce fragment a été muté de sorte que les nucléotides ATT codant pour une isoleucine en position 747 du récepteur sauvage (56) sont remplacés par les nucléotides ACC, codant pour une thréonine. Ce fragment muté a été cloné dans le vecteur pCre-ER (28) digéré par XhoI et Pstl, dont les extrémités ont également été remplies par la T4 polymérase [plasmide pCre+GR(I747/T)+F; séquence codante du LBD de hGR(I747/T) dans la même orientation que celle de Cre]. pCre-LBD[GR(I747/T)] a été obtenu par mutagénèse dirigée réalisée sur l'ADN simple brin préparé à partir du plasmide pCre + GR(I747/T) + F, avec les oligonucléotides de synthèse :
- SG52 (5'-CTGGAAGATGGCGATCTCGAGATTCAGCAGGCCACT-3')
- SG53 (5' -CTGTTTCATCAAAAGGGTACCAGCCGTGGAGGGGCAT- 3')
permettant de fusionner d'une part la séquence d'ADN codant pour la Cre [amino acides (aa) 1-343 (orf2 (cre) ; (31)], et celle qui code pour la région contenant le LBD muté de HG1 [aa 500-777], d'autre part la séquence d'ADN précédemment modifiée et celle codant pour la région F du récepteur humain des oestrogènes (ER) [aa 553-595 (57)] (Figure 5). Cette opération restaure le site de restriction XhoI situé à la fin des séquences Cre dans pCre-ER et introduit un site de restriction Kpnl entre les séquences codant pour GR et la région F du ER (Figures 6 et 7).

### 1.2. Mutation du gène RXRα

Le vecteur (pRXRα^{(LNL)}), utilisé pour muter le gène RXRα (58) par recombinaison homologue dans les cellules F9 a été construit de la façon suivante :

Un phage λ contenant un fragment génomique de 11.8 kb, comprenant les exons 2,3 et 4 de RXRα, a été isolé à partir d'une banque génomique réalisée avec de l'ADN génomique de l'embryocarcinome murin P19, cloné dans le vecteur λ EMBL3. Un fragment SalI isolé de ce phage (dont les extrémités ont été remplies par la T4 polymérase) a été sous cloné dans le site BamHI (après traitement à la T4 polymérase) d'un dérivé du vecteur pBluescriptIISK+ (Stratagène). Dans ce dérivé, le site XhoI a été supprimé. Un site XhoI a été introduit dans l'exon 4 de RXRα au niveau du site AccI correspondant au nucléotide 535 de l'ADNc de RXRα (la séquence de l'ADNc de RXRα est donnée dans Genbank référence : M 84817), en clonant dans le site AccI les oligonucléotides synthétiques suivants :
- 5'-ATTATTATTACTCGAGTGATGATG-3' et,
- 5'-ATCATCATCATCCGAGTAATAATA-3'.
Enfin, le fragment XhoI, contenant la cassette d'expression du gène de résistance à la néomycine, entourée de sites loxP, isolé du vecteur pHR56 (28) a été cloné dans le site XhoI du vecteur précédent.

La mutation d'un allèle du gène RXRα dans des ellules F9 a été effectuée par recombinaison homologue à l'aide du vecteur pRXRα^{(LNL)} (28). Cette lignée a été appelée RXRα^{+/-(LNL)}.

### 1.3. Expression de Cre-LBD[GR(I747/T)] dans des cellules comportant un fragment d'ADN modifié : RXRα^{(LNL)}

- Transfection transitoire : 5. 106 cellules RXRα^{+/-(LNL)} ou cellules HeLa resuspendues dans 500 µl de PBS ont été électroporées avec 5 µg de plasmide pCre-LBD[GR(I747/T)] [éventuellement cotransfectées avec 5 µg du vecteur pRXRα^{(LNL)} pour les cellules Hela] à l'aide d'un électroporateur (Bio-Rad gene pulser), réglé à 200 V et 960 µF. Les cellules ont ensuite été ensemensées à 10⁶ cellules par boîte de 100 mm et traitées, si nécessaire, avec des ligands.
- Transfection stable : 5. 106 cellules RXRα^{+/-(LNL)}, resuspendues dans 500 µl de PBS ont été électroporées avec 5 µg de pCre-LBD[GR(I747/T)] et 1 µg de pPGK-Hyg (59), digérés respectivement par SalI et PvuII, à l'aide d'un électroporateur (Bio-Rad gene pulser), réglé à 200 V et 960 µF. Les clones résistants ont été obtenus selon la procédure décrite par Metzger et al. (28).

### 1.4. Amplification par "PCR" d'une région d'ADN génomique localisée dans l'exon 4 de RXRα

Les amplifications d'ADN ont été réalisées en utilisant la technique de "Polymérase Chain Reaction" (PCR), selon le protocole décrit par Chen and Evans (60). Les amorces utilisées sont : SB211 et PZ105, ayant respectivement comme séquence nucléotidique 5'-GGCAAACACTATGG-3' et 5'-TTGCGTACTGTCCTCTT-3'. Après dénaturation pendant 8 min à 94°C, 2,5 unités de Taq polymérase sont ajoutées. L'amplification est effectuée en réalisant 35 cycles (30 sec. à 94°C, 30 sec. à 50°C) suivi d'un cycle (30 sec. à 94°C, 30 sec. à 50°C, 5 min à 72°C) dont le produit est conservé à 4°C.

Les produits des réactions ont été séparés sur gel de polyacrylamide (10 %) ou d'agarose (2,5%). L'ADN a été visualisé par UV suite à une coloration au bromure d'éthidium, ou par Southern Blot (42).

### 1.5. Test d'excision des séquences situées entre les sites LoxP

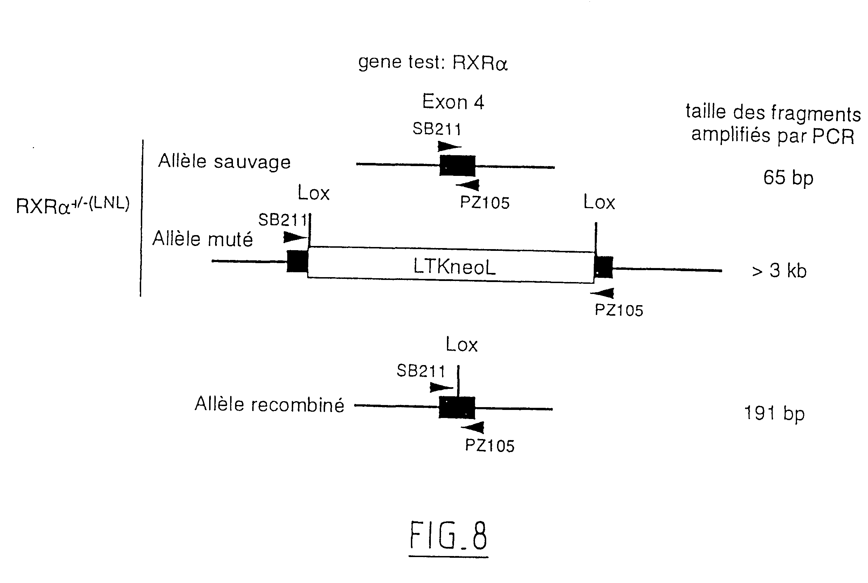

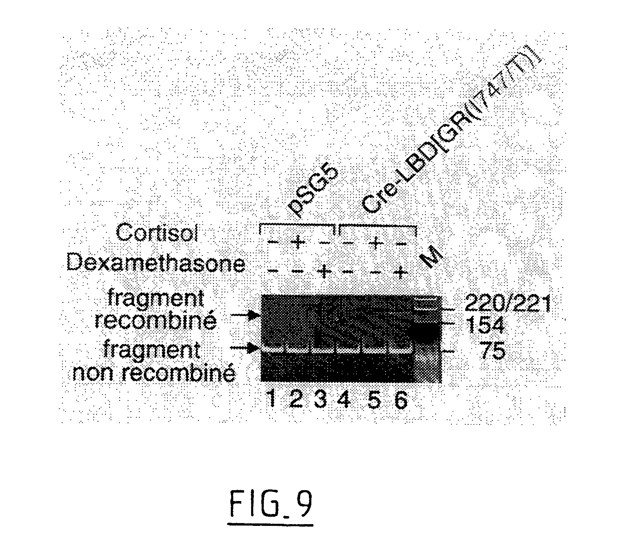

L'ADN de cellules RXRα^{+/-(LNL)}, transfectées avec pCre-LBD[GR(I747/T)] ou le vecteur parental pSG5, non traitées ou traitées avec du cortisol (10⁻⁶M) ou de la dexaméthasone (10⁻⁶M) pendant 72 heures, a été soumis a une réaction PCR selon la stratégie décrite en Figure 8. Les produits de la réaction ont été séparés sur gel d'acrylamide et colorés au bromure d'ethidium. La piste M sur la Figure 9 correspond au dépôt du marqueur de taille pBR322 digéré par Hinfl. La position des allèles sauvage et recombiné est indiquée.

### 1.6. Analyse par PCR de l'activité recombinase de Cre-LBD[GR(I747/T)] dans la lignée RXRα^{+/-(LNL)} exprimant la protéine chimérique de façon constitutive.

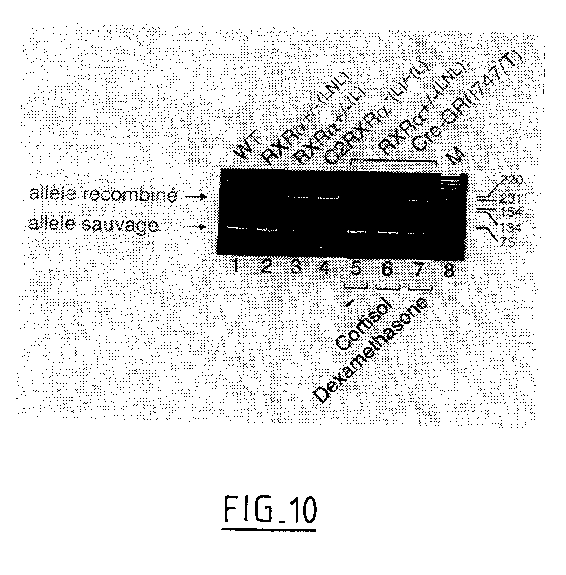

Pour la Figure 10, l'ADN préparé à partir de cellules RXRα^{+/-}-(LNL):Cre-GR(I747/T) non traitées ou traitées au cortisol (10⁻⁶M) ou à la dexamethasone (10⁻⁶M) a servi de matrice pour réaliser une amplification par PCR, selon la procédure décrite en Figure 8. L'ADN amplifié a été séparé sur gel d'acrylamide et coloré au bromure d'ethidium. Les pistes 1, 2, 3 et 4 contiennent respectivement les produits de PCR réalisés avec de l'ADN extrait de cellules F9 sauvages (WT), RXRα^{+/-(LNL)}, RXRα^{+/-(L)} et de C2RXRα^{-(L)/-(L)} (73). La piste 8 contient un marqueur de taille (échelle de 1kb ; GibcoBRL).

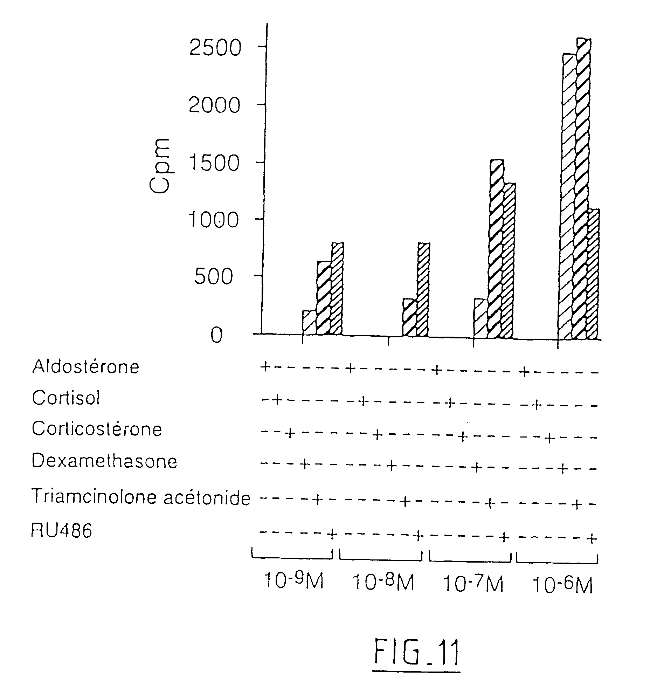

Sur la Figure 11, l'ADN préparé à partir de cellules RXRα^{+/-(LNL)}:Cre-GR(I747/T) non traitées ou traitées avec de l'aldostérone, du cortisol, de la corticostérone, de la dexamethasone, de la triamcinolone acetonide ou du RU486, à des concentrations allant de 10⁻⁹ à 10⁻⁶M, a servi de matrice pour réaliser une amplification par PCR, selon la procédure décrite en Figure 8. L'ADN amplifié a été séparé sur gel d'agarose et analysé selon la technique de Southern. Les fragments amplifiés correspondant aux allèles sauvage et recombiné ont été détectés par hybridation à l'aide de l'oligonucléotide de synthèse (5'-AAGAAGCCCTTGCAGCCCTC-3'), radiomarqué à l'extrémité 5' par de la T4 polynucléotide kinase. Le signal a été visualité et quantifié à l'aide d'un "phospho-imager" (Fujix bas2000).

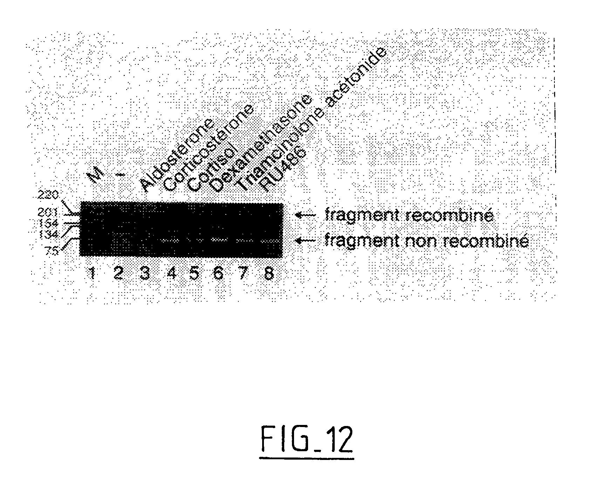

Pour la Figure 12, des cellules RXRα^{+/-(LNL)}:Cre-GR(I747/T) ont été cultivées pendant 72 h dans du milieu de culture auquel ont été ajoutés trois ligands naturels (l'aldostérone, le cortisol et la corticostérone) à la concentration de 10⁻⁹ M ou 10-8 M et de la dexaméthasone (10⁻⁷ ou 10-6 M) comme indiqué. L'allèle recombiné a été détecté selon la procédure décrite en Figure 8. L'ADN amplifié a été séparé sur gel d'agarose et analysé selon la technique Southern. Les fragments amplifiés correspondant à l'allèle recombiné ont été détectés par autoradiographie, suite à une hybridation avec l'oligonucléotide de synthèse
5'-AATTATAACTTCGTATAATGTATGCTATACGAAGTTATTC - 3', (contenant un site loxP) radiomarqué au Phosphore 32 à l'extrémité 5' par la T4 polynucléotide kinase.

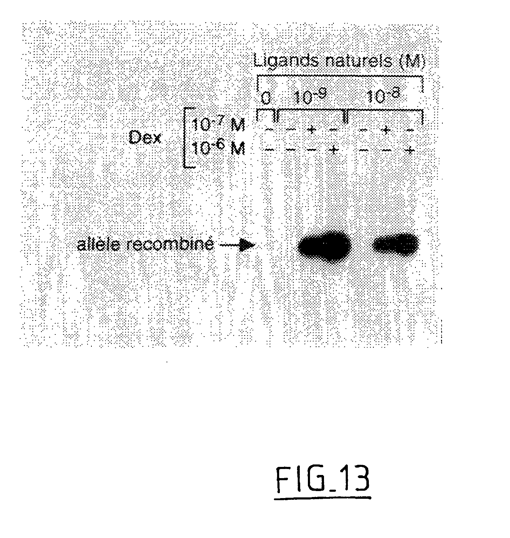

Pour la Figure 13, des cellules HeLa ont été cotransfectées par les plasmides pRXRα^{(LNL)} et pCre-LBD[GR(I747/T)], puis cultivées pendant 72 heures avec ou sans addition des ligands indiqués, à la concentration de 10⁻⁷M. L'ADN de ces cellules a ensuite été soumis à une amplification PCR selon la stratégie décrite. Les produits de la réaction ont été séparés sur gel d'acrylamide et colorés au bromure d'éthidium. La piste 1 correspond au dépôt du marqueur de taille 1 kb ladder (échelle de 1kb, GibcoBRL).

### 2. Résultats

Afin d'exprimer une recombinase Cre dont l'activité soit inductible par des ligands synthétiques du récepteur des glucocorticoïdes, mais non par des ligands glucocorticoïdes naturels aux concentrations "physiologiques", on a construit un vecteur d'expression, pCre-LBD[GR(I747/T)], dérivé du vecteur pCre-ER (28) en remplaçant la séquence d'ADN codant pour les acides aminés 344-618 contenant le LBD du récepteur des oestrogènes (RE), par celle codant pour les acides aminés 500-777 d'un récepteur des glucocorticoïdes (GR) muté contenant le domaine de liaison (LBD) muté du GR(I747/T). La construction de ce plasmide a conduit à l'insertion de la séquence GGTACC, codant pour les aa Gly-Thr en position 624-625 de Cre-LBD[GR(I747/T)] (Figure 7).

L'activité recombinase de Cre-LBD[GR(I747/T)] a été testée dans une lignée cellulaire murine RXRα^{+/-(LNL)}. Cette lignée a été obtenue en intégrant par recombinaison homologue le gène TK-neo, encadré de sites loxP (en répétition directe) (LNL), dans l'exon 4 d'un allèle du gène RXRα (28). Le gène TK-neo est un gène chimérique permettant l'expression d'une protéine de fusion thymidine kinase/gène de résistance à la néomycine. L'électroporation de cette lignée avec le vecteur pCre-LBD[GR(I747/T)] a permis d'évaluer son activité recombinase sans ajout de ligand, ou après addition de cortisol (ligand naturel) ou de dexamethasone (Figure 9) (Dex ; ligand synthétique). L'ADN des cellules ainsi traitées est en effet soumis à une PCR avec des oligonucléotides synthétiques localisés dans l'exon 4, l'un en amont du site AccI, l'autre en aval de ce site. La taille des fragments PCR obtenus avec cette paire d'oligonucléotides, est de 65 pb pour l'allèle sauvage, de 3345 pb pour l'allèle muté (après intégration de LNL) et de 191 pb pour l'allèle recombiné (après excision des séquences situées entre les deux sites lox et d'un des sites lox, voir figure 8). Suite à un traitement des cellules transfectées à la dexaméthasone (10⁻⁶ M), un fragment de 191 pb est observé, reflétant la présence de séquences correspondant à l'allèle recombiné, alors qu'en l'absence de traitement ou suite à un traitement au cortisol (10⁻⁶ M), aucun fragment de cette taille n'est observé. Ces résultats montrent donc que l'activité recombinase de la protéine de fusion étudiée peut être induite en traitant des cellules exprimant cette protéine avec un ligand synthétique du GR tel que la dexamethasone mais pas avec un ligand naturel tel que le cortisol, à la concentration de 10-6 M.

On a également établi une lignée RXRα^{+/-(LNL)} exprimant de façon constitutive Cre-LBD[GR(I747/T)], en cointégrant la cassette d'expression du gène Cre-LBD[GR(I747/T)] avec un vecteur exprimant le gène de résistance à l'hygromycine (RXRα^{+/-(LNL)} : Cre-GR(I747/T) ; voir "Matériel et Méthodes" ; données non présentées). L'activité recombinase a été testée suite à un traitement de ces cellules avec de la Dex (10⁻⁶M) pendant 72 heures. L'analyse des produits des PCR effectuées sur l'ADN de ces cellules révèle un fragment de 191 pb correspondant à l'allèle recombiné uniquement après traitement à la Dex (Figure 10).

Ainsi, il est montré que Cre-LBD[GR(I747/T)] est inactive sans ajout de ligand ou en présence de cortisol à 10-6 M, mais active en présence de Dex, lorsque sa cassette d'expression est intégrée dans le génome.

On a également évalué la cinétique d'action de la recombinase : son activité est détectable dès 12h de traitement à la dexaméthasone à 10⁻⁶ M, mais elle est beaucoup plus forte après 72h de traitement (résultats non présentés).

L'activité recombinase de Cre-LBD[GR(I747/T)] a aussi été testée suite à l'addition de différentes concentrations de ligands. Aucune activité recombinase n'a pu être observée dans les cellules RXRα^{+/-(LNL)}:Cre-GR(I747/T) traitées à l'aldostérone, au cortisol ou à la corticostérone (concentration des ligands allant de 10⁻⁹ à 10⁻⁶ M), alors qu'en présence de dex, de triamcinolone acetonide ou de RU486, une activité recombinase est observée dès 10⁻⁹ M (Figure 11).

On a également montré à l'aide de cette lignée cellulaire que l'activité recombinase de Cre-LBD[GR(I747/T)] peut être induite par des ligands synthétiques, même lorsque les cellules sont cultivées en présence de ligands naturels (aldostérone, cortisol et corticostérone) à 10-9 ou 10-8 M. L'excision de séquences localisées entre les sites loxP est obtenue suite à un traitement de 72h à la dexaméthasone à 10⁻⁷ M ou 10-6 M (Figure 12).

L'activité recombinase de Cre-LBD[GR(I747/T)] a enfin été testée dans des cellules humaines : des cellules HeLa ont été cotransfectées transitoirement avec les vecteurs pCre-LBD[GR(I747/T)] et pRXRα^{(LNL)}, puis cultivées soit sans ajout de ligand, soit en présence des ligands indiqués sur la Figure 13. Comme dans les cellules F9, une bande de 191 pb, correspondant au fragment recombiné, est amplifiée à partir d'ADN isolé des cellules traitées avec des ligands synthétiques (Dexamethasone, Triamcinolone acetonide ou RU486 à 10⁻⁷ M), alors que sans ajout de ligand, ou en présence de ligands naturels tels que l'aldostérone, la corticostérone ou le cortisol aux mêmes concentrations, seul le fragment correspondant à l'allèle sauvage peut être détecté.

Ces résultats montrent donc que l'activité recombinase de la protéine de fusion Cre-LBD[GR(I747/T)] est induite par des ligands synthétiques du récepteur des glucocorticoïdes, alors qu'elle est insensible aux ligands naturels tels que le cortisol, la corticostérone ou l'aldostérone, même à des concentrations aussi élevées que 10-6 M. De plus, la présence de ligands naturels n'empêche pas l'induction de l'activité recombinase de Cre-LBD[GR(I747/T)] par les ligands synthétiques.

### EXEMPLE 3 : Induction de l'activité d'une recombinase Cre chimérique chez la souris

### 1. Matériel et Méthodes

### 1.1. Construction de plasmides et établissement de souris transgéniques

Le vecteur pCMVCre-LBD[GR(I747/T)] a été construit en clonant le fragment EcoRI de 2 kb isolé de pCre-LBD[GR(I747/T)] dans le site EcoRI de pMGSV1 (60). Le fragment PvuII de 4,64 kb isolé de pCMVCre-LBD[GR(I747/T)] a été injecté dans des zygotes F1 (C57BL/6 X SJL) à une concentration de 4 ng/µl afin de produire des souris transgéniques selon la procédure classique (61).

### 1.2. Conditions de PCR

Les amplifications par PCR ont été réalisées dans une solution tampon contenant 10 mM Tris-HCl (pH = 8.8), 50 mM KCl, 1,5 mM MgCl2, 0,2 mM dNTPs, 0,25 µM de chaque amorce, 2 unités de Taq polymérase et 1 µg d'ADN génomique comme matrice. Après 30 cycles (30 sec à 94°C, 30 sec à 55°C, 1 min à 72°C) puis 1 cycle (30 sec à 90°C, 30 sec à 55°C, 5 min à 72°C), les produits d'amplification ont été séparés sur gel d'agarose à 2.2 % coloré au bromure d'ethidium.

### 1.3. Analyse du génotype des souris.

Le transgène Cre-LBD[GR(I747/T)] et les allèles de type sauvage et excisé RXRα^{ΔAF2(LNL)} ont été détectés par PCR. La détection du transgène Cre-LBD[GR(I747/T)] a été effectuée à l'aide des amorces nucléotidiques 5'-ATCCGAAAAGAAAACGTTGA-3' et 5'-ATCCAGGTTACGGATATAGT-3', celle des allèles du gène RXRα grâce aux amorces 5'-GGTTCTCCGGCCGCTTGGGT-3' et 5'-GAAGGCGATGCGCTGCGAAT-3'. De la même façon, l'excision du marqueur tk-neo encadré de sites loxP a été suivie par PCR à l'aide des amorces A (5'-CAAGGAGCCTCCTTTCTCTA-3') et B (5'-CCTGCTCTACCTGGTGACTT-3'). Ces amorces amplifient un fragment d'ADN de 156 paires de bases à partir de l'allèle de type sauvage et un fragment de 190 paires de bases à partir de l'allèle excisé RXRα^{ΔAF2(L)}.

### 2. Résultats

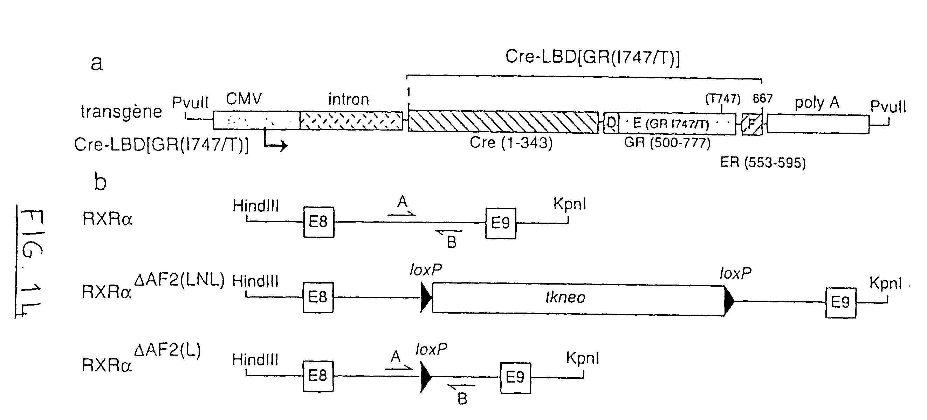

L'activité recombinase de Cre-LBD[GR(I747/T)] a également été testée chez la souris. Ainsi, des souris transgéniques exprimant la protéine de fusion Cre-LBD[GR(I747/T)] sous le contrôle du promoteur du gène "major IE" du cytomegalovirus human ont été établies. La structure du transgène est représentée à la figure 14a. Il contient les séquences activatrices/promotrices du gène "major IE" du cytomegalovirus humain, un intron du gène de la β-globine de lapin, les séquences codantes de Cre-LBD[GR(I747/T)] et le signal de polyadénylation du virus simien SV40. Le site d'initiation de la transcription est indiqué par une flèche. Ces souris ont ensuite été croisées avec des souris 'reporter', afin d'y démontrer l'existence d'une activité recombinase induite. Cette lignée 'reporter' contient une insertion du marqueur de selection tk-neo, encadré de sites loxP, dans l'intron situé entre les exons 8 et 9 du gène RXRα, sur l'un de ses deux allèles [RXRα^{ΔAF2(LNL)}]. Après recombinaison du marqueur tkneo, un site loxP reste en place et constitue l'allèle excisé RXRα^{ΔAF2(L)}. L'allèle RXRα de type sauvage et l'allèle excisé peuvent être détectés simultanement par amplification PCR en utilisant une paire d'amorce appropriée A et B. La figure 14b représente schématiquement cette stratégie PCR par indication des amorces A et B ainsi que de certains sites de restriction.

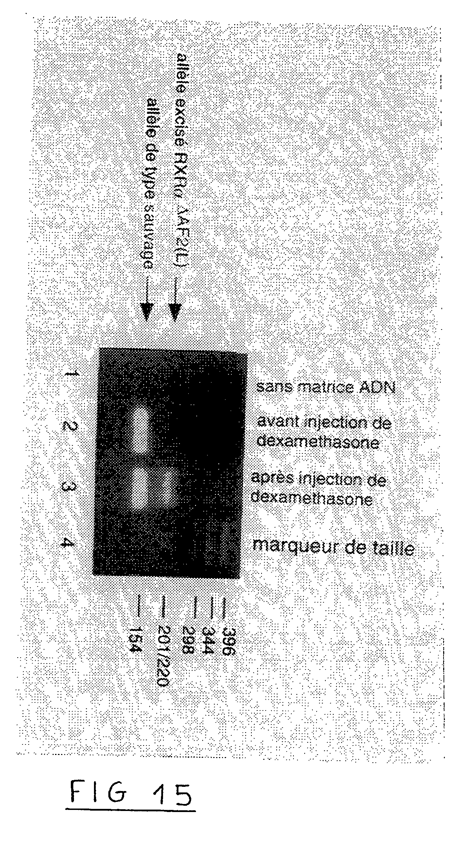

Les descendants issus du croisement de souris Cre-LBD[GR(I747/T)] et de souris 'reporter', décrites ci-dessus, contenant à la fois le transgène Cre-LBD[GR(I747/T)] et l'allèle RXRα^{ΔAF2(LNL)}, ont été identifiés en établissant leur génopype à partir d'une biopsie de queue. Une de ces souris a subi à l'âge de quatre semaines une injection intrapéritonéale quotidienne de 0.3 mg de dexaméthasone diluée dans 100 µl d'huile végétale, pendant cinq jours. Une biopsie de queue a été prélevée le jour avant la première injection, ainsi que le lendemain de la cinquième injection. L'ADN isolé à partir de ces prélèvements a été analysé par PCR à l'aide des oligonucléotides A et B (figure 15, pistes 2 et 3) pour déterminer si le traitement induit la délétion du marqueur tk-neo. La piste 1 correspond à une réaction contrôle, réalisée sans ADN. La position des produits de PCR amplifiés à partir de l'allèle RXRα de type sauvage et de l'allèle excisé RXRα^{ΔAF2(L)} sont indiqués. Le marqueur de taille (piste 4) correspond à l'échelle de 1 kb (GibcoBRL). La taille des fragments est donnée en paires de bases. D'après la figure 15, l'excision induite a bien eu lieu après traitement de la souris testée à la dexamethasone. En effet, l'analyse des fragments d'ADN amplifiés à partir de la queue avant traitement ne révèle que la présence de l'allèle RXRα de type sauvage, alors que l'analyse de la queue de la même souris, après traitement, indique la présence de l'allèle excisé RXRα^{ΔAF2(LNL)} (figure 15). Ainsi, il apparaît que la recombinase chimérique Cre-LBD[GR(I747/T)] ne présente aucune activité constitutive de base en présence des ligands endogènes. Elle est cepedant induite et active chez la souris par un traitement à la dexamethasone.

### REFERENCES

**1.** Evans RM, 1988, The steroid and thyroid hormone receptor superfamily. Science 240:889-895.
**2.** Beato M., 1989, Gene regulation by steroid hormones. Cell 56:335-344.
**3.** Green S, Chambon P., 1988, Nuclear receptors enhance our understanding of transcription regulation. Trends Genetique 4:309-314.
**4.** Parker MG, 1993, Steroid and related receptors. Curr. Opin. Cell. Biol. 5:499-504.
**5.** Simons SSJr, 1994, Function/activity of specific amino acids in glucocorticoid receptors, New York. Vitam. Horm. 49:49-130.
**6.** Danielian PS, WHite R, Lees JA, Parker MG, 1992, Identification of a conserved region required for hormone dependent transcriptional activation by steroid hormone receptors. EMBO J. 11:1025-1033.
**7.** Saatcioglu F., Bartunek P., Deng T., Zenke M., Karin M., 1993, A conserved C-terminal sequence that is deleted in v-ErbA is essential for the biological activities of c-ErbA (the thyroid hormone receptor). Mol. Cell. Biol. 13:3675-3685.
**8.** Barettino D., Vivanco Ruiz MdM., Stunnenberg HG., 1994, Characterization of the ligand-dependent transactivation domain of thyroid hormone receptor. EMBO J. 13:3039-3049.
**9.** Durand B., Saunders M., Gaudon C., Roy B., Losson R., Chambon P., 1995, Activation function 2 (AF-2) of retinoic acid receptor and 9-cis retinoic acid receptor: presence of a conserved autonomous constitutive activating domain and influence of the nature of the response element on AF-2 activity. EMBO J. 13:5370-5382.
**10**. Leng XH., Blanco J., Tsai SY., Ozato K., O'Malley BW., 1995, Mouse retinoid X receptor contains a separable ligand-binding and transactivation domain in its E region. Mol. Cell. Biol. 15:255-263.
11. Cavaillès V., Dauvois S., Danielian PS., Parker MG., 1994, Interaction of protein with transcriptionally active estrogen receptors, Proc. Natl. Acad. Sci. USA 91:10009-10013.
12. Halachmi S., Marden E., Martin G., MacKay H., Abbondanza C., Brown M., 1994, Estrogen receptor-associated proteins: possible mediators of hormone-induced transcription. Science 264:1455-1458.
13. Le Douarin B., Zechel C., Garnier JM., Lutz Y., Tora L, Pierrat B., Heery D., Gronemeyer H., Chambon P., Losson R. 1995, The N-terminal part of TIFI, a purative mediator of the ligand-dependent activation function (AF-2) of nuclear receptors, is fused to B-raf in the oncogenic protein T18. EMBO J. 14:2020-2033.
14. Cavaillès V., Dauvois S., L'Horset F., Lopez G., Hoare S., Kushner PJ., Parker MG., 1995, Nuclear factor RIP 140 modulates transcriptional activation by the estrogen receptor. EMBO J. 14:3741-3751.
15. Lee JW., Ryan F., Swaffield J., Johnston SA., Moore DD., 1995, Interaction of thyroid-hormone receptor with a conserved transcriptional mediator. Nature 374:91-94.
16. Baur et al., EMBO J., Vol. 15 n° 1, 110-124, 1996.
17. Bourguet W., Ruff P., Chambon P., Gronemeyer H., Moras D., 1995, Crystal structure of the ligand-binding domain of the human nuclear receptor RXR-α. Nature 375:377-382.
18. Renaud et al., Nature, Vol. n° 378 681-689, 1995.
**19.** Wurtz et al., Natural Structural Biology, Vol. 3, 87-93, 1996.
**20.** Chakraborti PK., Garebedian MJ., Yamamoto KR., Simons SSJ., 1991, Creation of "super" glucocorticoid receptors by point mutations in the steroid binding domain. J. Biol. Chem. 266:22075-22078.
**21.** Warriar N., Yu C., Govindan MV., 1994, Hormone binding domain of human glucocorticoid receptor enhancement of transactivation function by substitution mutants M565R and A573Q. J. Biol. Chem. 269:29010-29015.
**22.** Chen D., Kohli K., Zhank S., Danielsen M., Stallcup MR., 1994 Phenylalamine-780 near the C-terminus of the mouse glucocorticoid receptor is important for ligand binding affinity and specificity. Mol. Endocrinol. 8:422-430.
**23.** Hurley DM., Accili D., Stratakis CA., Karl M., Vamvakopoulos N., Rorer E., Constantine K., Taylor SI., Chrousos GP., 1991, Point mutation causing a single amino acid substitution in the hormone binding domain of the glucocorticoid receptor in familial glucocorticoid resistance. J. Clin. Invest. 87:680-686.
**24.** Keightley MC., Fuller PJ., 1994, Unique sequences in the Guinea Pig glucocorticoid receptor induce constitutive transactivation and decrease steroid sensitivity. Mol. Endocrinol. 8:431-439.
**25.** Chen D., Stallcup MR., 1994, The hormone-binding role of 2 cysteines near the C terminus of the mouse glucocorticoid receptor. J. Biol. Chem. 269:7914-1978.
**26.** Byravan S., Milhon J., Rabindran SK., Olinger B., Garabedian MJ., Danielsen M., Stallcup MR., 1991, Two point mutations in the hormone-binding domain of the mouse glucocorticoid receptor that dramatically reduce its function. Mol. Endocrinol. 5:752-758.
**27.** Picard, Current opinion in biotechnology, 1994, 5:511515.
**28.** Metzger et al., PNAS.USA Vol 92, July 1995, Genetics, 6991-6995.
**29.** Logie et al., PNAS USA Vol 92, June 1995, Genetics, 5940-5944.
**30.** Littlewood et al., Nucleic Acids Research 1995, Vol. 23, N° 10, 1686-1690.
**31.** Sternberg et al., J. Mol. Biol. (1986), 187:197-212.
**32.** Sauer et Henderson, The New Biologist, Vol. 2, N° 5, (May 1990), 441-449.
**33.** Barbonis et al. , Nucleic Acids Research, 1993, Vol. 21, N°9, 2025-2029.
**34.** Kilby et al., TIG, December 1993, Vol. 9, 413-421.
**35.** Sauer, Current opinion in Biotechnology, 1994, 5:521-527.
**36.** Denisen et al., PNAS USA Vol 92, (August 1995), Genetics, 7376-7380,
**37.** C. Babinet, Médecine/Sciences, 1995, n° 8 Vol. 11, 1154-1157.
**38.** Malchoff DM., Brufsky A., Reardon G., McDermott P., Javier EC., Bergh CH., Rowe D., Malchoff CD., 1993, A mutation of the glucocorticoid receptor in primary cortisol resistance. J. Clin. Invest. 91:1918-1925.
**39.** J.C. Kaplan et M. Delpech, Biologie Moléculaire et Médecine, 2ème Edition, Edition Flammarion.
**40.** Kumar V., Green S., Stack G., Berry M., Jin JR., Chambon P., 1987, Functional domains of the human estrogen receptor. Cell. 51:941-951.
**41.** Green S., Issemann I., Sheer E., 1988, A versatile in vivo and in vitro eukaryotic expression vector for protein engineering. Nucl. Acids. Res. 16:369.
**42.** Sambrook J., Fritsch EF., Maniatis T., 1989, Molecular cloning. A laboratory manual. In: eds. Cold Spring Harbor, New York: Cold Spring Harbor Laboratory Press.
**43.** Webster NJG., Green S., Jin JR., Chambon P., 1988, The hormone-bnding domains of the estrogen and glucocorticoid receptors contain an inducible transcription activation function. Cell. 54:199-207.
**44.** Lanz RB., Rusconi S., 1994, A conserved carboxy-terminal subdomain is important for ligand interpretation and transactivation by nuclear receptors. Endocrinology 135:2183-2195.
**45.** Mahfoudi A., Roulet E., Dauvois S., Parker MG., Wahli W., 1995, Specific mutations in the estrogen receptor change the properties of antiestrogens to full agonists. Proc. Natl. Acad. Sci. USA 92:4206-4210.
**46.** Vegeto E., Allan GF., Schrader WT., Tsai MJ., McDonnel DP., O'Malley BW., 1992, The mecanism of RU486 antagonism is dependent on the conformation of the carboxy-terminal tail of the human progesterone receptor. Cell. 69:703-713.
**47.** McKnight S., Tjian R. 1986, Transcriptional selectivity of viral genes in Mammalian cells. Cell. 46:795-805.
**48.** Schwartz O., VIrelizier JL., Montagnier L., Hazan U., 1990, A microtransfection method using the luciferase-encoding reporter gene for the assay of human immunodeficiency virus LTR promoter activity. Gene 88:197-205.
**49.** Bocquel MT., Kumar V., Stricker C., Chambon P., Gronemeyer H., 1989, The contribution of the N- and C-terminal regions of steroid receptors to activation of transcription is both receptor and cell-specific. NucI. Acids. Res. 17:2581-2595.
**50.** Grange T., Roux J., Rigaud G., Pictet R., 1989, Two remote glucocorticoid responsive units interact cooperatively to promote glucocorticoid induction of rat tyrosine aminotransferase gene expression. Nucl. Acids. Res. 17:8695-8709.
**51.** Pons M., Gagne D., Nicolas JC., Mehtali M., 1990, A new cellular model of response to estrogens : a bioluminescent test to characterize (anti)estrogen molecules. Bio Techniques 9:450-459.
**52.** Green S., Kumar V., Theulaz I., Wahli W., Chambon P., 1988, The N-terminal 'zinc finger' of the oestrogen and glucocorticoid receptors determines target gene specificity. EMBO J. 7:3037-3044.
**53.** De Wet JR., Wood KV., De Luca M., Helinski DR., Subramani S., 1987, Firefly luciferase gene : structure and expression in mammalian cells. Mol. Cell. Biol. 7:725-737.
**54.** Jausons-Loffreda N., Balaguer P., Roux S., Fuentes M., Pons M., Nicolas JC., Gelmini S., Pazzagli M., 1994, Chimeric receptors as a tool for luminiscent measurement of biological activities of steroid hormones. Jour. Bioluminescence and Chemiluminescence 9:217-221.
**55.** Kakidanj H., Ptashne M., 1988, Gal4 activated gene expression in mammalian cells. Cell. 52:161-167.
**56.** Hollenberg SM., Weinberger C., Ong ES., Cerelli G., Oro A., Lebo R., Thompson EB., Rosenfeld MG., Evans RM. 1985, Primary structure and expression of a human glucocorticoid receptor cDNA. Nature 318:635-641.
**57.** Green et al., 1986, Nature 320, 134-139.
**58.** Mangelsdorf D.J., Borgmeyer V., Heyman R.A., Zhow JY., Ony E.S., Oro A.E, Kakizuka A and Evans R.M., 1992, Genes and Development 6, 329-344.
**59.** Riele et al., 1990, Nature 348, 649-651.60. Chen and Evans (1993) (Methods in molecular biology, Vol 15 : PCR protocols current methods and applications chap. 7, Edited by : BA White Copyright, 1993, Humana Press Inc. Totowa, NJ).
**60.** R. Feil, J. Brocard, B. Mascrez, M. LeMeur, D. Metzger, P. Chambon. (1996) Ligand-activated site-specific recombination in mice. Proc. Natl. Acad. Sci. U. S. A., 93, 10887-10890.
**61.** Hogan, B., Costantini, F., & Lacy, E. (1986) Manipulating the Mouse Embryo: A Laboratory Manual (Cold Spring Harbor Lab., Cold Spring Harbor, NY).

## Revendications

1. Fragment d'ADN codant pour un récepteur nucléaire des glucocorticoïdes sauvage avec une mutation isoleucine --> thréonine située entre les hélices H11 et H12 de la séquence en acides aminés du récepteur humain sauvage, de sorte que l'activité dudit récepteur est inductible plus fortement par un ligand glucocorticoïde synthétique que par un ligand glucocorticoïde naturel.

2. Fragment d'ADN selon la revendication 1, **caractérisé en ce qu'**il a pour séquence, une séquence codant pour le récepteur nucléaire humain des glucocorticoïdes dont la séquence en acides aminés est substantiellement telle que représentée dans l'IDS n°1.

3. Fragment d'ADN selon la revendication 2, **caractérisé en ce qu'**il a pour séquence la séquence de l'ADNc du récepteur nucléaire humain des glucocorticoïdes substantiellement telle que représentée dans l'IDS n°1.

4. Fragment d'ADN codant pour le domaine de liaison du ligand (LBD) du récepteur nucléaire humain des glucocorticoïdes sauvage avec une mutation isoleucine --> thréonine en position correspondant à la position 747 de la séquence en acides aminés dudit récepteur complet.

5. Fragment d'ADN codant pour le domaine de liaison du ligand du récepteur nucléaire humain des glucocorticoïdes selon la revendication 4, **caractérisé en ce qu'**il a pour séquence, une séquence codant pour les acides aminés du domaine de liaison du ligand du récepteur nucléaire humain des glucocorticoïdes dont la séquence en acides aminés est substantiellement telle que représentée dans l'IDS n°1 de l'acide aminé 532 à l'acide aminé 777.

6. Fragment d'ADN selon la revendication 5, **caractérisé en ce qu'**il a pour séquence la séquence codant pour le domaine de liaison du ligand (LBD) du récepteur nucléaire humain des glucocorticoïdes substantiellement telle que représentée dans l'IDS n°1 du codon 532 au codon 777.

7. Récepteur nucléaire des glucocorticoïdes modifié, ledit récepteur modifié ayant pour séquence, la séquence en acides aminés du récepteur humain sauvage avec une mutation isoleucine --> thréonine située entre les hélices H11 et H12, de sorte que l'activité dudit récepteur est inductible plus fortement par un ligand glucocorticoïde synthétique que par un ligand glucocorticoïde naturel.

8. Récepteur nucléaire des glucocorticoïdes selon la revendication 7, **caractérisé en ce qu'**il a pour séquence en acides aminés, une séquence substantiellement telle que représentée dans l'IDS n°1.

9. Domaine de liaison du ligand du récepteur nucléaire des glucocorticoïdes selon la revendication 7 ou 8.

10. Domaine de liaison du ligand du récepteur nucléaire humain des glucocorticoïdes LBD [GR(I747/T)], **caractérisé en ce qu'**il a pour séquence, la séquence en acides aminés du domaine de liaison du ligand du récepteur des glucocorticoïdes humain substantiellement telle que représentée dans l'IDS n°1 de l'acide aminé 532 à l'acide aminé 777.

11. Système vectoriel d'expression conditionnelle d'une protéine notamment une protéine étrangère dans des cellules hôtes comportant un élément de contrôle de la transcription inductible par un complexe formé par un récepteur nucléaire des glucocorticoïdes et un ligand, **caractérisé en ce qu'**il comprend :
- un premier fragment d'ADN consistant en un fragment d'ADN codant pour ladite protéine sous le contrôle d'éléments assurant son expression dans lesdites cellules hôtes, lesdits éléments assurant son expression comprenant une séquence de contrôle de transcription (RE) inductible par le récepteur selon la revendication 7 ou 8, complexé à un ligand glucocorticoïde synthétique, et
- un deuxième fragment d'ADN consistant en un fragment d'ADN fonctionnel codant pour ledit récepteur selon l'une des revendications 1 à 3 ou seulement une partie dudit fragment comprenant la région qui reconnaît le ligand (LBD) selon l'une des revendications 4 à 6, et la région de liaison à l'ADN (DBD) qui se fixe sur ladite séquence de contrôle de la transcription (RE),
- lesdits premier et deuxième fragments d'ADN pouvant être portés par un même vecteur ou deux vecteurs séparément.

12. Système vectoriel selon la revendication 11, **caractérisé en ce qu'**il comporte l'ADNc codant pour :
- la région LBD, représentée par des codons codant les acides aminés n° 532 à 777 dans l'IDS n°1, et
- la région DBD, représentée par des codons codant les acides aminés n° 421 à 487 dans l'IDS n°1.

13. Système vectoriel selon la revendication 11, **caractérisé en ce qu'**on remplace la séquence codant pour le domaine de liaison à l'ADN (DBD) du récepteur des glucocorticoïdes par celle d'un autre transactivateur, notamment celui de la protéine de levure Gal 4 et on remplace la séquence de contrôle de transcription (RE) du récepteur des glucocorticdides par celle reconnue par ledit autre transactivateur, notamment la séquence 17m reconnue par le transactivateur Gal 4.

14. Procédé d'expression d'une protéine étrangère dans des cellules humaines ou animales notamment de mammifères, **caractérisé en ce que** l'on cultive des cellules qui contiennent un système vectoriel selon l'une des revendications 11 à 13, et **en ce qu'**on ajoute ledit ligand synthétique, dans le milieu de culture, puis on récupère la protéine synthétisée.

15. Procédé selon la revendication 14, **caractérisé en ce que** ledit ligand synthétique est choisi parmi la dexaméthasone, le triamcinolone acétonide, le RU28362, le bimétrazole, le déacylcortivazol et le fluocinolone acetonide.

16. Gène de fusion comprenant un fragment d'ADN selon l'une des revendications 1 à 6 et un fragment d'ADN codant pour une protéine dont on veut réguler l'activité, ladite activité étant inductible plus fortement par liaison dudit récepteur ou dudit domaine de liaison du ligand du récepteur avec un ligand glucocorticoïde synthétique qu'avec un ligand glucocorticdide naturel, lorsque ladite protéine se trouve fusionnée audit récepteur ou audit domaine de liaison du ligand dudit récepteur.

17. Gène de fusion selon la revendication 16, **caractérisé en ce que** ladite protéine est une protéine recombinase.

18. Gène de fusion selon la revendication 17, **caractérisé en ce que** ladite protéine est une recombinase de la famille des intégrases λ.

19. Gène de fusion selon la revendication 18, **caractérisé en ce que** la protéine recombinase est la recombinase Cre du bactériophage P1.

20. Gène de fusion selon la revendication 19, comprenant un fragment d'ADN codant pour la protéine recombinase Cre du bactériophage P1, et le fragment d'ADN codant pour le domaine de liaison du ligand du récepteur nucléaire humain des glucocorticoïdes modifié selon l'une des revendications 1 à 6.

21. Gène de fusion selon la revendication 20, comprenant dans le sens 5' --> 3' :
- un fragment d'ADN codant pour la recombinase Cre du bactériophage P1 ;
- un fragment d'ADN codant pour tout ou partie de la région charnière D du récepteur nucléaire des glucocorticoïdes, région située entre le domaine DBD et le domaine LBD, et
- le fragment d'ADN codant pour le domaine de liaison du ligand (LBD) modifié, notamment muté, du récepteur nucléaire des glucocorticoïdes selon l'une des revendications 1 à 6.

22. Gène de fusion selon la revendication 21, **caractérisé en ce qu'**il a pour séquence une séquence codant pour les acides aminés de l'IDS n°2 comprenant :
- les acides aminés 1 à 343 qui correspondent à la recombinase Cre ;
- les acides aminés 346 à 377 qui correspondent à la région C- terminale de la région D du récepteur humain des glucocorticoïdes;
- les acides aminés 378 à 623 qui correspondent au LBD [GR(I747/T)].

23. Gène de fusion selon la revendication 22, **caractérisé en ce qu'**il a substantiellement la séquence Cre-LBD[GR(I747/T)] telle que représentée dans l'IDS n°2 dont les acides aminés 626 à 667 correspondent à la région F du récepteur humain des oestrogènes.

24. Vecteur d'expression de la protéine de fusion codée par le gène de fusion selon l'une des revendications 16 à 23 dans des cellules hôtes animales notamment de mammifères, **caractérisé en ce qu'**il comporte le gène de fusion selon l'une des revendications 16 à 23, placé sous le contrôle d'éléments d'expression assurant son expression dans lesdites cellules hôtes.

25. Protéine de fusion comprenant un récepteur selon la revendication 7 ou 8 ou un domaine de liaison du ligand selon la revendication 9 ou 10 et une protéine dont l'activité est inductible plus fortement par liaison dudit récepteur ou dudit domaine de liaison du ligand (LBD) avec un ligand glucocorticoïde synthétique qu'avec un ligand glucocorticoïde naturel.

26. Protéine de fusion selon la revendication 25, **caractérisée en ce que** la protéine est une protéine recombinase.

27. Protéine de fusion selon la revendication 25, **caractérisée en ce que** la protéine est une protéine recombinase de la famille des intégrases λ.

28. Protéine de fusion selon la revendication 27, **caractérisée en ce que** la recombinase est la protéine Cre du bactériophage P1.

29. Protéine de fusion selon la revendication 28, comportant en outre la partie C- terminale de la région charnière D du récepteur nucléaire humain des glucocorticoïdes, intercalée entre la protéine Cre et ledit domaine de liaison du ligand du récepteur modifié selon la revendication 9 ou 10.

30. Procédé de recombinaison, notamment, d'excision, insertion, inversion ou translocation conditionnelle au niveau d'un fragment d'ADN contenant un ou deux sites de reconnaissance spécifique d'une protéine recombinase dans des cellules hôtes animales, **caractérisé en ce qu'**il comporte les étapes dans lesquelles:
1) on introduit une protéine de fusion selon l'une des revendications 26 à 29 ou un vecteur d'expression de ladite proteine de fusion selon la revendication 24 dans lesdites cellules hôtes dans des conditions permettant l'expression d'une protéine de fusion selon l'une des revendications 26 à 29, et
2) on complexe ladite protéine de fusion avec un ligand glucocorticoïde synthétique, en mettant ledit ligand en présence desdites cellules hôtes.

31. Procédé de délétion conditionnelle d'un fragment d'ADN dans un gène dans lequel on met en oeuvre un procédé d'excision selon la revendication 30, et dans lequel ledit fragment d'ADN à exciser est intégré entre deux sites de reconnaissance de protéine recombinase orientés en répétition directe.

32. Procédé selon la revendication 30 ou 31, **caractérisé en ce que** lesdits sites de reconnaissance spécifique d'une protéine recombinase sont les sites loxP et ladite protéine recombinase est la protéine Cre du bactériophage P1.

33. Procédé selon l'une des revendications 30 à 32, **caractérisé en ce que** ledit fragment d'ADN que l'on veut exciser et lesdits sites de reconnaissance spécifiques d'une protéine recombinase sont portés par un vecteur plasmidique ou viral, ou intégrés dans un chromosome des cellules hôtes.

34. Procédé selon l'une des revendications 30 à 33, **caractérisé en ce que** l'intégration des sites de reconnaissance spécifique de la protéine recombinase, notamment du ou des sites loxP, se fait par recombinaison homologue du gène comportant ledit fragment d'ADN à insérer ou transloquer ou respectivement à exciser ou inverser avec un gène modifié comportant ledit fragment d'ADN flanqué en 5' et/ou 3' par le ou lesdit(s) site(s) de reconnaissance de recombinase, notamment site(s) loxP.

35. Vecteur de transfert d'un fragment d'ADN dans des cellules hôtes humaines ou animales, notamment mammifères, **caractérisé en ce qu'**il comporte un fragment d'ADN à transférer comprenant des sites de reconnaissance spécifiques de la recombinase, notamment deux sites loxP orientés à répétition directe et une cassette d'expression d'un gène de fusion selon l'une des revendications 16 à 23.

36. Vecteur selon la revendication 35, **caractérisé en ce que** le gène de fusion est placé sous le contrôle d'éléments d'expression spécifiques des cellules hôtes.

37. Vecteur selon la revendication 36, **caractérisé en ce qu'**il comporte la séquence du plasmide pCre-LBD[GR(I747/T)] de l'IDS n°3.

38. Procédé selon l'une des revendications 30 à 34, **caractérisé en ce que** ledit fragment d'ADN à exciser a été transféré, notamment intégré dans le chromosome desdites cellules, à l'aide d'un vecteur de transfert selon la revendication 35.

39. Vecteur selon la revendication 24 ou 35 pour une utilisation comme médicament en thérapie génique en combinaison avec un ligand glucocorticoïde synthétique, notamment la dexaméthasone.

40. Cellule humaine in vitro ou animale, notamment de mammifères transfectée par un vecteur d'expression d'une protéine de fusion selon la revendication 24.

41. Cellule humaine in vitro ou animale, dans laquelle un fragment d'ADN a été transféré à l'aide d'un vecteur, selon la revendication 35 ou 36.

42. Cellule animale in vitro, notamment de mammifères exprimant de façon constitutive la protéine de fusion selon l'une des revendications 25 à 29.

43. Animal transgénique à l'exclusion de l'humain, notamment souris, comportant un gène fonctionnel de la protéine de fusion selon l'une des revendications 25 à 29 notamment intégré dans un de ses chromosomes.

44. Animal transgénique selon la revendication 43 dans lequel un gène d'intérêt est modifié par insertion de un ou plusieurs site(s) loxP, notamment intégré(s) dans un ou plusieurs de ses chromosomes.

45. Utilisation d'un vecteur, de cellules ou d'un animal à l'exclusion de l'humain selon l'une des revendications 42 à 44, comme outil d'analyse ou étude de fonctionnement d'un gène donné d'une cellule hôte.

46. Méthode de traitement des cellules *ex vivo* ou *in vitro* impliquant la mise en oeuvre d'un procédé selon l'une des revendications 30 à 34 ou 38 et l'utilisation d'un vecteur selon la revendication 39.

## Patentansprüche

1. DNA-Fragment, welches einen nukleären Rezeptor der Glucocorticoide vom Wildtyp mit einer Isoleucin→Threonin-Mutation, welche sich zwischen den Helices H11 und H12 der Aminosäuresequenz des humanen Rezeptors vom Wildtyp befindet, kodiert derart, dass die Aktivität des Rezeptors durch einen synthetischen Glucocorticoid-Liganden stärker induzierbar ist als durch einen natürlichen Glucocorticoid-Liganden.

2. DNA-Fragment nach Anspruch 1, **dadurch gekennzeichnet, dass** es als Sequenz eine Sequenz aufweist, die den humanen nukleären Rezeptor der Glucocorticoide, dessen Aminosäuresequenz im Wesentlichen diejenige ist, die in der IDS Nr. 1 angegeben ist, kodiert.

3. DNA-Fragment nach Anspruch 2, **dadurch gekennzeichnet, dass** es als Sequenz die cDNA-Sequenz des humanen nukleären Rezeptors der Glucocorticoide, die im Wesentlichen diejenige ist, die in der IDS Nr. 1 angegeben ist, aufweist.

4. DNA-Fragment, welches die Ligandenbindungsdomäne (LBD) des humanen nukleären Rezeptors der Glucocorticoide vom Wildtyp mit einer Isoleucin→Threonin-Mutation an einer Position, welche der Position 747 der Aminosäuresequenz des vollständigen Rezeptors entspricht, kodiert.

5. DNA-Fragment, welches die Ligandenbindungsdomäne des humanen nukleären Rezeptors der Glucocorticoide kodiert, nach Anspruch 4, **dadurch gekennzeichnet, dass** es als Sequenz eine Sequenz aufweist, die die Aminosäuren der Ligandenbindungsdomäne des humanen nukleären Rezeptors der Glucocorticoide, deren Aminosäuresequenz im Wesentlichen diejenige ist, die in der IDS Nr. 1 von Aminosäure 532 bis Aminosäure 777 angegeben ist, kodiert.

6. DNA-Fragment nach Anspruch 5, **dadurch gekennzeichnet, dass** es als Sequenz die Sequenz aufweist, die die Ligandenbindungsdomäne (LBD) des humanen nukleären Rezeptors der Glucocorticoide, die im Wesentlichen diejenige ist, die in der IDS Nr. 1 von Codon 532 bis Codon 777 angegeben ist, kodiert.

7. Modifizierter nukleärer Rezeptor der Glucocorticoide, wobei der modifizierte Rezeptor als Sequenz die Aminosäuresequenz des humanen Wildtyp-Rezeptors mit einer Isoleucin→Threonin-Mutation, welche sich zwischen den Helices H11 und H12 befindet, derart, dass die Aktivität des Rezeptors durch einen synthetischen Glucocorticoid-Liganden stärker induzierbar ist als durch einen natürlichen Glucocorticoid-Liganden, aufweist.

8. Nukleärer Rezeptor der Glucocorticoide nach Anspruch 7, **dadurch gekennzeichnet, dass** er als Aminosäuresequenz eine Sequenz, die im Wesentlichen diejenige ist, die in der IDS Nr. 1 angegeben ist, aufweist.

9. Ligandenbindungsdomäne des nukleären Rezeptors der Glucocorticoide nach Anspruch 7 oder 8.

10. Ligandenbindungsdomäne des humanen nukleären Rezeptors der Glucocorticoide LBD [GR(I747/T)], **dadurch gekennzeichnet, dass** sie als Sequenz die Aminosäuresequenz der Ligandenbindungsdomäne des humanen Rezeptors der Glucocorticoide, die im Wesentlichen diejenige ist, die in der IDS Nr. 1 von Aminosäure 532 bis Aminosäure 777 angegeben ist, aufweist.

11. Vektorsystem zur konditionalen Expression eines Proteins, insbesondere eines fremden Proteins in Wirtszellen, umfassend ein Transkriptionskontrollelement, welches durch einen Komplex, welcher durch einen nukleären Rezeptor der Glucocorticoide und einen Liganden gebildet wird, induzierbar ist, **dadurch gekennzeichnet, dass** es umfasst:
- ein erstes DNA-Fragment, welches aus einem DNA-Fragment besteht, welches das Protein kodiert, unter der Kontrolle von Elementen, die dessen Expression in den Wirtszellen sicherstellen, wobei die Elemente, die dessen Expression sicherstellen, eine Transkriptionskontrollsequenz (RE), welche durch den Rezeptor nach Anspruch 7 oder 8, welcher mit einem synthetischen Glucocorticoid-Liganden komplexiert ist, induzierbar ist, umfassen, und
- ein zweites DNA-Fragment, welches aus einem funktionsfähigen, den Rezeptor kodierenden DNA-Fragment nach einem der Ansprüche 1 bis 3 oder nur einem Teil des Fragments, umfassend die Region, die den Liganden erkennt (LBD), nach einem der Ansprüche 4 bis 6 und der DNA-Bindungsregion (DBD), die an die Transkriptionskontrollsequenz (RE) bindet, besteht,
- wobei das erste und das zweite DNA-Fragment sich auf ein und demselben Vektor oder getrennt auf zwei Vektoren befinden können.

12. Vektorsystem nach Anspruch 11, **dadurch gekennzeichnet, dass** es die cDNA umfasst, welche kodiert für:
- die LBD-Region, welche durch Codons angegeben wird, welche die Aminosäuren Nr. 532 bis 777 in der IDS Nr. 1 kodieren, und
- die DBD-Region, welche durch Codons angegeben wird, welche die Aminosäuren Nr. 421 bis 487 in der IDS Nr. 1 kodieren.

13. Vektorsystem nach Anspruch 11, **dadurch gekennzeichnet, dass** man die die DNA-Bindungsdomäne (DBD) des Rezeptors der Glucocorticoide kodierende Sequenz durch jene eines anderen Transaktivators, insbesondere jene des Hefe-Proteins Gal4, ersetzt und man die Transkriptionskontrollsequenz (RE) des Rezeptors der Glucocorticoide durch jene, die durch den anderen Transaktivator erkannt wird, insbesondere die Sequenz 17m, die durch den Transaktivator Gal 4 erkannt wird, ersetzt.

14. Verfahren zur Expression eines fremden Proteins in humanen oder tierischen Zellen, insbesondere von Säugetieren, **dadurch gekennzeichnet, dass** man Zellen, die ein Vektorsystem nach einem der Ansprüche 11 bis 13 enthalten, kultiviert und dass man den synthetischen Liganden in das Kulturmedium hinzusetzt und man dann das synthetisierte Protein gewinnt.

15. Verfahren nach Anspruch 14, **dadurch gekennzeichnet, dass** der synthetische Ligand aus Dexamethason, Triamcinolon-acetonid, RU28362, Bimetrazol, Deacylcortivazol und Fluocinolon-acetonid ausgewählt wird.

16. Fusioniertes Gen, umfassend ein DNA-Fragment nach einem der Ansprüche 1 bis 6 und ein DNA-Fragment, welches ein Protein kodiert, dessen Aktivität man zu regulieren wünscht, wobei die Aktivität stärker induzierbar ist durch Bindung eines synthetischen Glucocorticoid-Liganden als eines natürliche Glucocorticoid-Liganden an den Rezeptor oder die Ligandenbindungsdomäne des Rezeptors, wenn das Protein sich an den Rezeptor oder die Ligandenbindungsdomäne des Rezeptors fusioniert befindet.

17. Fusioniertes Gen nach Anspruch 16, **dadurch gekennzeichnet, dass** das Protein ein Rekombinase-Protein ist.

18. Fusioniertes Gen nach Anspruch 17, **dadurch gekennzeichnet, dass** das Protein eine Rekombinase aus der Familie der λ-Integrasen ist.

19. Fusioniertes Gen nach Anspruch 18, **dadurch gekennzeichnet, dass** das Rekombinase-Protein die Rekombinase Cre des Bakteriophagen P1 ist.

20. Fusioniertes Gen nach Anspruch 19, umfassend ein DNA-Fragment, welches das Rekombinase-Protein Cre des Bakteriophagen P1 kodiert, und das DNA-Fragment, welches die Ligandenbindungsdomäne des modifizierten humanen nukleären Rezeptors der Glucocorticoide nach einem der Ansprüche 1 bis 6 kodiert.

21. Fusioniertes Gen nach Anspruch 20, umfassend in der 5'→3'-Richtung:
- ein DNA-Fragment, welches die Rekombinase Cre des Bakteriophagen P1 kodiert;
- ein DNA-Fragment, welches die Gesamtheit oder einen Teil der Gelenkregion D des nukleären Rezeptors der Glucocorticoide, einer Region, die sich zwischen der DBD-Domäne und der LBD-Domäne befindet, kodiert, und
- das DNA-Fragment, welches die modifizierte, insbesondere mutierte Ligandenbindungsdomäne (LBD) des nukleären Rezeptors der Glucocorticoide nach einem der Ansprüche 1 bis 6 kodiert.

22. Fusioniertes Gen nach Anspruch 21, **dadurch gekennzeichnet, dass** es als Sequenz eine Sequenz aufweist, die die Aminosäuren der IDS Nr. 2 kodiert, umfassend:
- die Aminosäuren 1 bis 343, die der Rekombinase Cre entsprechen;
- die Aminosäuren 346 bis 377, die der C-terminalen Region der Region D des humanen Rezeptors der Glucocorticoide entsprechen;
- die Aminosäuren 378 bis 623, die der LBD [GR(I747/T)] entsprechen.

23. Fusioniertes Gen nach Anspruch 22, **dadurch gekennzeichnet, dass** es im Wesentlichen die Sequenz Cre-LBD[GR(I747/T)], wie sie in der IDS Nr. 2 angegeben ist, deren Aminosäuren 626 bis 667 der Region F des humanen Rezeptors der Östrogene entsprechen, aufweist.

24. Vektor für die Expression des Fusionsproteins, welches durch das fusionierte Gen nach einem der Ansprüche 16 bis 23 kodiert wird, in tierischen, insbesondere von Säugetieren stammenden Wirtszellen, **dadurch gekennzeichnet, dass** er das fusionierte Gen nach einem der Ansprüche 16 bis 23, welches unter die Kontrolle von Expressionselementen, welche dessen Expression in den Wirtszellen sicherstellen, gestellt ist, umfasst.

25. Fusionsprotein, umfassend einen Rezeptor nach Anspruch 7 oder 8 oder eine Ligandenbindungsdomäne nach Anspruch 9 oder 10 und ein Protein, dessen Aktivität stärker induzierbar ist durch Bindung eines synthetischen Glucocorticoid-Liganden als eines natürlichen Glucocorticoid-Liganden an den Rezeptor oder die Ligandenbindungsdomäne (LBD).

26. Fusionsprotein nach Anspruch 25, **dadurch gekennzeichnet, dass** das Protein ein Rekombinase-Protein ist.

27. Fusionsprotein nach Anspruch 25, **dadurch gekennzeichnet, dass** das Protein ein Rekombinase-Protein aus der Familie der λ-Integrasen ist.

28. Fusionsprotein nach Anspruch 27, **dadurch gekennzeichnet, dass** die Rekombinase das Protein Cre des Bakteriophagen P1 ist.

29. Fusionsprotein nach Anspruch 28, welches außerdem den C-terminalen Teil der Gelenkregion D des humanen nukleären Rezeptors der Glucocorticoide zwischengeschaltet zwischen das Protein Cre und die Ligandenbindungsdomäne des modifizierten Rezeptors nach Anspruch 9 oder 10 umfasst.

30. Verfahren zur konditionalen Rekombination, insbesondere Exzision, Insertion, Inversion oder Translokation auf der Höhe eines DNA-Fragments, welches ein oder zwei spezifische Erkennungsstellen für ein Rekombinase-Protein in tierischen Wirtszellen enthält, **dadurch gekennzeichnet, dass** es die Schritte umfasst, in welchen:
1) man ein Fusionsprotein nach einem der Ansprüche 26 bis 29 oder einen Expressionsvektor des Fusionsproteins nach Anspruch 24 in die Wirtszellen unter Bedingungen, die die Expression eines Fusionsproteins nach einem der Ansprüche 26 bis 29 erlauben, einführt und
2) man das Fusionsprotein mit einem synthetischen Glucocorticoid-Liganden komplexiert, indem man den Liganden in Gegenwart der Wirtszellen bringt.

31. Verfahren zur konditionalen Deletion eines DNA-Fragments in einem Gen, in welchem man ein Exzisionsverfahren nach Anspruch 30 ausführt und in welchem das herauszuschneidende DNA-Fragment zwischen zwei Erkennungsstellen für ein Rekombinase-Protein, die in direkter Wiederholung orientiert sind, integriert ist.

32. Verfahren nach Anspruch 30 oder 31, **dadurch gekennzeichnet, dass** die spezifischen Erkennungsstellen für ein Rekombinase-Protein die loxP-Stellen sind und das Rekombinase-Protein das Protein Cre des Bakteriophagen P1 ist.

33. Verfahren nach einem der Ansprüche 30 bis 32, **dadurch gekennzeichnet, dass** das DNA-Fragment, das man herauszuschneiden wünscht, und die spezifischen Erkennungsstellen für ein Rekombinase-Protein sich auf einem Plasmid-Vektor oder viralen Vektor befinden oder in ein Chromosom der Wirtszellen integriert sind.

34. Verfahren nach einem der Ansprüche 30 bis 33, **dadurch gekennzeichnet, dass** die Integration der spezifischen Erkennungsstellen für das Rekombinase-Protein, insbesondere der loxP-Stelle(n), erfolgt durch homologe Rekombination des Gens, welches das zu inserierende oder zu translozierende bzw. herauszuschneidende oder zu invertierende DNA-Fragment umfasst, mit einem modifizierten Gen, welches das DNA-Fragment, welches 5' und/oder 3' von der oder den Rekombinase-Erkennungsstelle(n), insbesondere der oder den loxP-Stelle(n) flankiert wird, umfasst.

35. Vektor für den Transfer eines DNA-Fragments in humane oder tierische Wirtszellen, insbesondere Säugetier-Wirtszellen, **dadurch gekennzeichnet, dass** er ein zu transferierendes DNA-Fragment, welches spezifische Erkennungsstellen für die Rekombinase, insbesondere zwei loxP-Stellen, die in direkter Wiederholung orientiert sind, umfasst, und eine Expressionskassette eines fusionierten Gens nach einem der Ansprüche 16 bis 23 umfasst.

36. Vektor nach Anspruch 35, **dadurch gekennzeichnet, dass** das fusionierte Gen unter die Kontrolle von spezifischen Expressionselementen der Wirtszellen gestellt ist.

37. Vektor nach Anspruch 36, **dadurch gekennzeichnet, dass** er die Sequenz des Plasmids pCre-LBD[GR(I747/T)] der IDS Nr. 3 umfasst.

38. Verfahren nach einem der Ansprüche 30 bis 34, **dadurch gekennzeichnet, dass** das herauszuschneidende DNA-Fragment transferiert, insbesondere in das Chromosom der Zellen integriert worden ist mit Hilfe eines Transfervektors nach Anspruch 35.

39. Vektor nach Anspruch 24 oder 35 für eine Verwendung als Arzneimittel bei einer Gentherapie in Kombination mit einem synthetischen Glucocorticoid-Liganden, insbesondere Dexamethason.

40. Humane oder tierische Zelle in vitro, insbesondere Säugetierzelle, welche durch einen Vektor zur Expression eines Fusionsproteins nach Anspruch 24 transfiziert ist.

41. Humane oder tierische Zelle in vitro, in welche ein DNA-Fragment mit Hilfe eines Vektors nach Anspruch 35 oder 36 transferiert worden ist.

42. Tierische Zelle in vitro, insbesondere Säugetierzelle, welche konstitutiv das Fusionsprotein nach einem der Ansprüche 25 bis 29 exprimiert.

43. Transgenes Tier mit Ausnahme des Menschen, insbesondere Maus, welche(s) ein funktionsfähiges Gen des Fusionsproteins nach einem der Ansprüche 25 bis 29, welches insbesondere in eines von dessen bzw. deren Chromosomen integriert ist, umfasst.

44. Transgenes Tier nach Anspruch 43, in welchem ein Gen von Interesse durch Insertion von einer oder mehreren loxP-Stelle(n), die insbesondere in ein oder mehrere von dessen Chromosomen integriert ist bzw. sind, modifiziert ist.

45. Verwendung eines Vektors, von Zellen oder eines Tiers mit Ausnahme des Menschen nach einem der Ansprüche 42 bis 44 als Tool zur Analyse oder Untersuchung der Funktion oder Funktionsweise eines gegebenen Gens einer Wirtszelle.

46. Verfahren zur Behandlung von Zellen *ex vivo* oder *in vitro,* welches das Ausführen eines Verfahrens nach einem der Ansprüche 30 bis 34 oder 38 und die Verwendung eines Vektors nach Anspruch 39 umfasst.

## Claims

1. DNA fragment coding for a wild-type nuclear glucocorticoid receptor with an isoleucine --> threonine mutation situated between helices H11 and H12 of the amino acid sequence of the wild-type human receptor, so that the activity of said receptor is more strongly inducible by a synthetic glucocorticoid ligand than by a natural glucocorticoid ligand.

2. DNA fragment according to Claim 1, **characterized in that** it has, as sequence, a sequence coding for the human nuclear glucocorticoid receptor whose amino acid sequence is substantially as represented in SID No. 1.

3. DNA fragment according to Claim 2, **characterized in that** it has, as sequence, the cDNA sequence of the human nuclear glucocorticoid receptor substantially as represented in SID No. 1.

4. DNA fragment coding for the ligand binding domain (LBD) of the wild-type human nuclear glucocorticoid receptor with an isoleucine --> threonine mutation at a position corresponding to position 747 of the amino acid sequence of said complete receptor.

5. DNA fragment coding for the ligand binding domain of the human nuclear glucocorticoid receptor according to Claim 4, **characterized in that** it has, as sequence, a sequence coding for the amino acids of the ligand binding domain of the human nuclear glucocorticoid receptor whose amino acid sequence is substantially as represented in SID No. 1 from amino acid 532 to amino acid 777.

6. DNA fragment according to Claim 5, **characterized in that** it has, as sequence, the sequence coding for the ligand binding domain (LBD) of the human nuclear glucocorticoid receptor substantially as represented in SID No. 1 from codon 532 to codon 777.

7. Modified nuclear glucocorticoid receptor, said modified receptor having, as sequence, the amino acid sequence of the wild-type human receptor with an isoleucine --> threonine mutation situated between helices H11 and H12, so that the activity of said receptor is more strongly inducible by a synthetic glucocorticoid ligand than by a natural glucocorticoid ligand.

8. Nuclear glucocorticoid receptor according to Claim 7, **characterized in that** it has, as amino acid sequence, a sequence substantially as represented in SID No. 1.

9. Ligand binding domain of the nuclear glucocorticoid receptor according to Claim 7 or 8.

10. Ligand binding domain of the human nuclear glucocorticoid receptor LBD [GR(I747/T)], **characterized in that** it has, as sequence, the amino acid sequence of the ligand binding domain of the human glucocorticoid receptor substantially as represented in SID No. 1 from amino acid 532 to amino acid 777.

11. Vector system for conditionally expressing a protein, in particular a foreign protein, in host cells comprising an element for control of transcription inducible by a complex formed by a nuclear glucocorticoid receptor and a ligand, **characterized in that** it comprises:
- a first DNA fragment consisting of a DNA fragment coding for said protein under the control of elements ensuring its expression in said host cells, said elements ensuring its expression comprising a sequence for control of transcription (RE) inducible by the receptor according to Claim 7 or 8 complexed with a synthetic glucocorticoid ligand, and
- a second DNA fragment consisting of a functional DNA fragment coding for said receptor according to one of Claims 1 to 3 or only part of said fragment comprising the region which recognizes the ligand (LBD) according to one of Claims 4 to 6, and the DNA binding region (DBD) which attaches to said sequence for control of transcription (RE),
- it being possible for said first and second DNA fragments to be carried by the same vector or two vectors separately.

12. Vector system according to Claim 11, **characterized in that** it comprises the cDNA coding for:
- the LBD region, represented by codons coding for amino acids Nos 532 to 777 in SID No. 1, and
- the DBD region, represented by codons coding for amino acids Nos 421 to 487 in SID No. 1.

13. Vector system according to Claim 11, **characterized in that** the sequence coding for the DNA binding domain (DBD) of the glucocorticoid receptor is replaced by that of another transactivator, in particular that of the yeast protein Gal 4 and the sequence for control of transcription (RE) of the glucocorticoid receptor is replaced by that recognized by said other transactivator, in particular the 17m sequence recognized by the Gal 4 transactivator.

14. Method of expressing a foreign protein in human or animal, in particular mammalian, cells, **characterized in that** cells are cultured which contain a vector system according to one of Claims 11 to 13, and **in that** said synthetic ligand is added to the culture medium and then the protein synthesized is recovered.

15. Method according to Claim 14, **characterized in that** said synthetic ligand is chosen from dexamethasone, triamcinolone acetonide, RU28362, bimetrazole, deacylcortivazol and fluocinolone acetonide.

16. Fusion gene comprising a DNA fragment according to one of Claims 1 to 6 and a DNA fragment coding for a protein of which it is desired to regulate the activity, said activity being more strongly inducible by binding of said receptor or of said ligand binding domain of the receptor with a synthetic glucocorticoid ligand than with a natural glucocorticoid ligand, when said protein is fused to said receptor or to said ligand binding domain of said receptor.

17. Fusion gene according to Claim 16, **characterized in that** said protein is a recombinase protein.

18. Fusion gene according to Claim 17, **characterized in that** said protein is a recombinase of the family of λ integrases.

19. Fusion gene according to Claim 18, **characterized in that** the recombinase protein is the Cre recombinase of bacteriophage P1.

20. Fusion gene according to Claim 19, comprising a DNA fragment coding for the Cre recombinase protein of bacteriophage P1, and the DNA fragment coding for the ligand binding domain of the modified human nuclear glucocorticoid receptor according to any of Claims 1 to 6.

21. Fusion gene according to Claim 20, comprising in the 5' --> 3' direction:
- a DNA fragment coding for the Cre recombinase of bacteriophage P1;
- a DNA fragment coding for all or part of the hinge region D of the nuclear glucocorticoid receptor, a region situated between the DBD domain and the LBD domain, and
- the DNA fragment coding for the modified, in particular mutated, ligand binding domain (LBD) of the nuclear glucocorticoid receptor according to any of Claims 1 to 6.

22. Fusion gene according to Claim 21, **characterized in that** it has, as sequence, a sequence coding for the amino acids of SID No. 2 comprising:
- amino acids 1 to 343 which correspond to the Cre recombinase;
- amino acids 346 to 377 which correspond to the C-terminal region of the D region of the human glucocorticoid receptor;
- amino acids 378 to 623 which correspond to the LBD [GR(I747/T)].

23. Fusion gene according to Claim 22, **characterized in that** it has substantially the sequence Cre-LBD[GR(I747/T)] as represented in SID No. 2 in which amino acids 626 to 667 correspond to the F region of the human oestrogen receptor.

24. Vector for expressing the fusion protein encoded by the fusion gene according to one of Claims 16 to 23 in animal, in particular mammalian, host cells, **characterized in that** it comprises the fusion gene according to one of Claims 16 to 23, placed under the control of elements for expression ensuring its expression in said host cells.

25. Fusion protein comprising a receptor according to Claim 7 or 8 or a ligand binding domain according to Claim 9 or 10 and a protein whose activity is more strongly inducible by the binding of said receptor or of said ligand binding domain (LBD) with a synthetic glucocorticoid ligand than with a natural glucocorticoid ligand.

26. Fusion protein according to Claim 25, **characterized in that** the protein is a recombinase protein.

27. Fusion protein according to Claim 25, **characterized in that** the protein is a recombinase protein of the family of λ integrases.

28. Fusion protein according to Claim 27, **characterized in that** the recombinase is the Cre protein of bacteriophage P1.

29. Fusion protein according to Claim 28, comprising, in addition, the C-terminal part of the hinge region D of the human nuclear glucocorticoid receptor, intercalated between the Cre protein and said ligand binding domain of the modified receptor according to Claim 9 or 10.

30. Method of recombination, in particular of conditional excision, insertion, inversion or translocation at the level of a DNA fragment containing one or two sites for specific recognition of a recombinase protein in animal host cells, **characterized in that** it comprises the steps in which:
1) a fusion protein according to one of Claims 26 to 29 or a vector for expressing said fusion protein according to Claim 24 is introduced into said host cells under conditions allowing the expression of a fusion protein according to one of Claims 26 to 29, and
2) said fusion protein is complexed with a synthetic glucocorticoid ligand by exposing said ligand to said host cells.

31. Method for the conditional deletion of a DNA fragment in a gene in which a method of excision according to Claim 30 is used, and in which said DNA fragment to be excised is integrated between two recombinase protein recognition sites oriented as a direct repeat.

32. Method according to Claim 30 or 31, **characterized in that** said recognition sites specific for a recombinase protein are the loxP sites and said recombinase protein is the Cre protein of bacteriophage P1.

33. Method according to one of Claims 30 to 32, **characterized in that** said DNA fragment which it is desired to excise and said recognition sites specific for a recombinase protein are carried by a plasmid or viral vector, or are integrated into a chromosome of the host cells.

34. Method according to one of Claims 30 to 33, **characterized in that** the integration of the recombinase protein specific recognition sites, in particular of the loxP site(s), takes place by homologous recombination of the gene comprising said DNA fragment to be inserted or translocated or respectively to be excised or inverted with a modified gene comprising said DNA fragment flanked in 5' and/or 3' by said recombinase recognition site(s), in particular the loxP site(s).

35. Vector for transferring a DNA fragment into human or animal, in particular mammalian, host cells, **characterized in that** it comprises a DNA fragment to be transferred comprising recognition sites specific for the recombinase, in particular two loxP sites, oriented as direct repeats and a cassette for expression of a fusion gene according to one of Claims 16 to 23.

36. Vector according to Claim 35, **characterized in that** the fusion gene is placed under the control of expression elements specific for the host cells.

37. Vector according to Claim 36, **characterized in that** it comprises the sequence of the plasmid pCre-LBD[GR(I747/T)] of SID No. 3.

38. Method according to one of Claims 30 to 34, **characterized in that** said DNA fragment to be excised was transferred, in particular integrated into the chromosome of said cells, with the aid of a transfer vector according to Claim 35.

39. Vector according to Claim 24 or 35 for use as a medicament in gene therapy in combination with a synthetic glucocorticoid ligand, in particular dexamethasone.

40. Human or animal, in particular mammalian, cell in vitro transfected with a vector for expressing a fusion protein according to Claim 24.

41. Human or animal cell in vitro, into which a DNA fragment has been transferred with the aid of a vector according to Claim 35 or 36.

42. Animal, in particular mammalian, cell in vitro constitutively expressing the fusion protein according to one of Claims 25 to 29.

43. Transgenic animal, excluding humans, in particular mice, comprising a functional gene for the fusion protein according to one of Claims 25 to 29, in particular integrated into one of its chromosomes.

44. Transgenic animal according to Claim 43, in which a gene of interest is modified by insertion of one or more loxP sites, in particular integrated into one or more of its chromosomes.

45. Use of a vector, of cells or of an animal, excluding humans, according to one of Claims 42 to 44, as a tool for analysing or studying the function of a given gene of a host cell.

46. Method of treating cells ex vivo or in vitro involving the use of a method according to one of Claims 30 to 34 or 38 and the use of a vector according to Claim 39.


### LISTE DE SEQUENCES

(1) INFORMATIONS GENERALES:
   (i) DEPOSANT:
      (A) NOM: ASSOCIATION POUR LE DEVELOPPEMENT DE LA RECHERCHE GENETIQUE MOLECULAIRE (ADEREGEM)
      (B) RUE: 26 RUE MONTPENSIER
      (C) VILLE: PARIS
      (E) PAYS: FRANCE
      (F) CODE POSTAL: 75001
   (ii) TITRE DE L' INVENTION: RECEPTEUR NUCLEAIRE DE GLUCOCORTICOIDES MODIFIE, PROTEINE DE FUSION, FRAGMENTS D'ADN CODANT POUR LEDIT RECEPTEUR ET LADITE PROTEINE DE FUSION, VECTEURS, CELLULES ET ANIMAUX EN COMPRENANT ET PROCEDES DANS LESQUELS ILS SONT MIS EN OEUVRE
   (iii) NOMBRE DE SEQUENCES: 17
   (iv) FORME DECHIFFRABLE PAR ORDINATEUR:
      (A) TYPE DE SUPPORT: Floppy disk
      (B) ORDINATEUR: IBM PC compatible
      (C) SYSTEME D' EXPLOITATION: PC-DOS/MS-DOS
      (D) LOGICIEL: Patentln Release #1.0, Version #1.30 (OEB)
(2) INFORMATIONS POUR LA SEQ ID NO: 1:
   (i) CARACTERISTIQUES DE LA SEQUENCE:
      (A) LONGUEUR: 2334 paires de bases
      (B) TYPE: nucléotide
      (C) NOMBRE DE BRINS: simple
      (D) CONFIGURATION: linéaire
   (ii) TYPE DE MOLECULE: ADNc
   (ix) CARACTERISTIQUE:
      (A) NOM/CLE: GR(I747/T) (Récepteur humain des glucocorticoïdes muté en position 747)
      (B) EMPLACEMENT:1..2331
   (i) CARACTERISTIQUES DE LA SEQUENCE:
      (A) LONGUEUR: 777 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 1:
(2) INFORMATIONS POUR LA SEQ ID NO: 2:
   (i) CARACTERISTIQUES DE LA SEQUENCE:
      (A) LONGUEUR: 2004 paires de bases
      (B) TYPE: nucléotide
      (C) NOMBRE DE BRINS: simple
      (D) CONFIGURATION: linéaire
   (ii) TYPE DE MOLECULE: ADNc
   (ix) CARACTERISTIQUE:
      (A) NOM/CLE: CRE-LBD(GR(I747/T) (Protéine de fusion du domaine LBD du récepteur humain des glucocorticoïdes muté en position 747 et de la protéine recombinase Cre)
      (B) EMPLACEMENT:1..2001
   (i) CARACTERISTIQUES DE LA SEQUENCE:
      (A) LONGUEUR: 667 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 2:
(2) INFORMATIONS POUR LA SEQ ID NO: 3
   (i) CARACTERISTIQUES DE LA SEQUENCE:
      (A) LONGUEUR: 6094 paires de bases
      (B) TYPE: nucléotide
      (C) NOMBRE DE BRINS: simple
      (D) CONFIGURATION: circulaire

   (ii) TYPE DE MOLECULE: ADNc
   (ix) CARACTERISTIQUE:
      (A) NOM/CLE:pCRE-LBD[GR(I747/T] (Plasmide d'expression de la protéine de fusion Cre-LBD[GR(I797/T)]
      (B) EMPLACEMENT:1..6091
   (xi) DESCRIPTION DE LA SEQUENCE: SEQ ID NO: 3:
   GTCGACTTCT GAGGCGGAAA GAACCAGCTG TGGAATGTGT GTCAGTTAGG GTGTGGAAAG 60
   TCCCCAGGCT CCCCAGCAGG CAGAAGTATG CAAAGCATGC ATCTCAATTA GTCAGCAACC 120
   AGGTGTGGAA AGTCCCCAGG CTCCCCAGCA GGCAGAAGTA TGCAAAGCAT GCATCTCAAT 180
   TAGTCAGCAA CCATAGTCCC GCCCCTAACT CCGCCCATCC CGCCCCTAAC TCCGCCCAGT 240
   TCCGCCCATT CTCCGCCCCA TGGCTGACTA ATTTTTTTTA TTTATGCAGA GGCCGAGGCC 300
   GCCTCGGCCT CTGAGCTATT CCAGAAGTAG TGAGGAGGCT TTTTTGGAGG CCTAGGCTTT 360
   TGCAAAAAGC TGGATCGATC CTGAGAACTT CAGGGTGAGT TTGGGGACCC TTGATTGTTC 420
   TTTCTTTTTC GCTATTGTAA AATTCATGTT ATATGGAGGG GGCAAAGTTT TCAGGGTGTT 480
   GTTTAGAATG GGAAGATGTC CCTTGTATCA CCATGGACCC TCATGATAAT TTTGTTTCTT 540
   TCACTTTCTA CTCTGTTGAC AACCATTGTC TCCTCTTATT TTCTTTTCAT TTTCTGTAAC 600
   TTTTTCGTTA AACTTTAGCT TGCATTTGTA ACGAATTTTT AAATTCACTT TTGTTTATTT 660
   GTCAGATTGT AAGTACTTTC TCTAATCACT TTTTTTTCAA GGCAATCAGG GTATATTATA 720
   TTGTACTTCA GCACAGTTTT AGAGAACAAT TGTTATAATT AAATGATAAG GTAGAATATT 780
   TCTGCATATA AATTCTGGCT GGCGTGGAAA TATTCTTATT GGTAGAAACA ACTACATCCT 840
   GGTCATCATC CTGCCTTTCT CTTTATGGTT ACAATGATAT ACACTGTTTG AGATGAGGAT 900
   AAAATACTCT GAGTCCAAAC CGGGCCCCTC TGCTAACCAT GTTCATGCCT TCTTCTTTTT 960
   CCTACAGCTC CTGGGCAACG TGCTGGTTAT TGTGCTGTCT CATCATTTTG GCAAAGAATT 1020
   GTAATACGAC TCACTATAGG GCGAATTCCA CCATGTCCAA TTTACTGACC GTACACCAAA 1080
   ATTTGCCTGC ATTACCGGTC GATGCAACGA GTGATGAGGT TCGCAAGAAC CTGATGGACA 1140
   TGTTCAGGGA TCGCCAGGCG TTTTCTGAGC ATACCTGGAA AATGCTTCTG TCCGTTTGCC 1200
   GGTCGTGGGC GGCATGGTGC AAGTTGAATA ACCGGAAATG GTTTCCCGCA GAACCTGAAG 1260
   ATGTTCGCGA TTATCTTCTA TATCTTCAGG CGCGCGGTCT GGCAGTAAAA ACTATCCAGC 1320
   AACATTTGGG CCAGCTAAAC ATGCTTCATC GTCGGTCCGG GCTGCCACGA CCAAGTGACA 1380
   GCAATGCTGT TTCACTGGTT ATGCGGCGGA TCCGAAAAGA AAACGTTGAT GCCGGTGAAC 1440
   GTGCAAAACA GGCTCTAGCG TTCGAACGCA CTGATTTCGA CCAGGTTCGT TCACTCATGG 1500
   AAAATAGCGA TCGCTGCCAG GATATACGTA ATCTGGCATT TCTGGGGATT GCTTATAACA 1560
   CCCTGTTACG TATAGCCGAA ATTGCCAGGA TCAGGGTTAA AGATATCTCA CGTACTGACG 1620
   GTGGGAGAAT GTTAATCCAT ATTGGCAGAA CGAAAACGCT GGTTAGCACC GCAGGTGTAG 1680
   AGAAGGCACT TAGCCTGGGG GTAACTAAAC TGGTCGAGCG ATGGATTTCC GTCTCTGGTG 1740
   TAGCTGATGA TCCGAATAAC TACCTGTTTT GCCGGGTCAG AAAAAATGGT GTTGCCGCGC 1800
   CATCTGCCAC CAGCCAGCTA TCAACTCGCG CCCTGGAAGG GATTTTTGAA GCAACTCATC 1860
   GATTGATTTA CGGCGCTAAG GATGACTCTG GTCAGAGATA CCTGGCCTGG TCTGGACACA 1920
   GTGCCCGTGT CGGAGCCGCG CGAGATATGG CCCGCGCTGG AGTTTCAATA CCGGAGATCA 1980
   TGCAAGCTGG TGGCTGGACC AATGTAAATA TTGTCATGAA CTATATCCGT AACCTGGATA 2040
   GTGAAACAGG GGCAATGGTG CGCCTGCTGG AAGATGGCGA TCTCGAGATT CAGCAGGCCA 2100
   CTACAGGAGT CTCACAAGAA ACCTCTGAAA ATCCTGGTAA CAAAACAATA GTTCCTGCAA 2160
   CGTTACCACA ACTCACCCCT ACCCTGGTGT CACTGTTGGA GGTTATTGAA CCTGAAGTGT 2220
   TATATGCAGG ATATGATAGC TCTGTTCCAG ACTCAACTTG GAGGATCATG ACTACGCTCA 2280
   ACATGTTAGG AGGGCGGCAA GTGATTGCAG CAGTGAAATG GGCAAAGGCA ATACCAGGTT 2340
   TCAGGAACTT ACACCTGGAT GACCAAATGA CCCTACTGCA GTACTCCTGG ATGTTTCTTA 2400
   TGGCATTTGC TCTGGGGTGG AGATCATATA GACAATCAAG TGCAAACCTG CTGTGTTTTG 2460
   CTCCTGATCT GATTATTAAT GAGCAGAGAA TGACTCTACC CTGCATGTAC GACCAATGTA 2520
   AACACATGCT GTATGTTTCC TCTGAGTTAC ACAGGCTTCA GGTATCTTAT GAAGAGTATC 2580
   TCTGTATGAA AACCTTACTG CTTCTCTCTT CAGTTCCTAA GGACGGTCTG AAGAGCCAAG 2640
   AGCTATTTGA TGAAATTAGA ATGACCTACA TCAAAGAGCT AGGAAAAGCC ATTGTCAAGA 2700
   GGGAAGGAAA CTCCAGCCAG AACTGGCAGC GGTTTTATCA ACTGACAAAA CTCTTGGATT 2760
   CTATGCATGA AGTGGTTGAA AATCTCCTTA ACTATTGCTT CCAAACATTT TTGGATAAGA 2820
   CCATGTCCAC CGAGTTCCCC GAGATGTTAG CTGAAATCAT CACCAATCAG ATACCAAAAT 2880
   ATTCAAATGG AAATATCAAA AAACTTCTGT TTCATCAAAA GGGTACCAGC CGTGGAGGGG 2940
   CATCCGTGGA GGAGACGGAC CAAAGCCACT TGGCCACTGC GGGCTCTACT TCATCGCATT 3000
   CCTTGCAAAA GTATTACATC ACGGGGGAGG CAGAGGGTTT CCCTGCCACA GTCTGAGAGC 3060
   TCCCTGGAAT TCGGATCTTA TTAAAGCAGA ACTTGTTTAT TGCAGCTTAT AATGGTTACA 3120
   AATAAAGCAA TAGCATCACA AATTTCACAA ATAAAGCATT TTTTTCACTG CATTCTAGTT 3180
   GTGGTTTGTC CAAACTCATC AATGTATCTT ATCATGTCTG GTCGACTCTA GACTCTTCCG 3240
   CTTCCTCGCT CACTGACTCG CTGCGCTCGG TCGTTCGGCT GCGGCGAGCG GTATCAGCTC 3300
   ACTCAAAGGC GGTAATACGG TTATCCACAG AATCAGGGGA TAACGCAGGA AAGAACATGT 3360
   GAGCAAAAGG CCAGCAAAAG GCCAGGAACC GTAAAAAGGC CGCGTTGCTG GCGTTTTTCC 3420
   ATAGGCTCCG CCCCCCTGAC GAGCATCACA AAAATCGACG CTCAAGTCAG AGGTGGCGAA 3480
   ACCCGACAGG ACTATAAAGA TACCAGGCGT TTCCCCCTGG AAGCTCCCTC GTGCGCTCTC 3540
   CTGTTCCGAC CCTGCCGCTT ACCGGATACC TGTCCGCCTT TCTCCCTTCG GGAAGCGTGG 3600
   CGCTTTCTCA TAGCTCACGC TGTAGGTATC TCAGTTCGGT GTAGGTCGTT CGCTCCAAGC 3660
   TGGGCTGTGT GCACGAACCC CCCGTTCAGC CCGACCGCTG CGCCTTATCC GGTAACTATC 3720
   GTCTTGAGTC CAACCCGGTA AGACACGACT TATCGCCACT GGCAGCAGCC ACTGGTAACA 3780
   GGATTAGCAG AGCGAGGTAT GTAGGCGGTG CTACAGAGTT CTTGAAGTGG TGGCCTAACT 3840
   ACGGCTACAC TAGAAGGACA GTATTTGGTA TCTGCGCTCT GCTGAAGCCA GTTACCTTCG 3900
   GAAAAAGAGT TGGTAGCTCT TGATCCGGCA AACAAACCAC CGCTGGTAGC GGTGGTTTTT 3960
   TTGTTTGCAA GCAGCAGATT ACGCGCAGAA AAAAAGGATC TCAAGAAGAT CCTTTGATCT 4020
   TTTCTACGGG GTCTGACGCT CAGTGGAACG AAAACTCACG TTAAGGGATT TTGGTCATGA 4080
   GATTATCAAA AAGGATCTTC ACCTAGATCC TTTTAAATTA AAAATGAAGT TTTAAATCAA 4140
   TCTAAAGTAT ATATGAGTAA ACTTGGTCTG ACAGTTACCA ATGCTTAATC AGTGAGGCAC 4200
   CTATCTCAGC GATCTGTCTA TTTCGTTCAT CCATAGTTGC CTGACTCCCC GTCGTGTAGA 4260
   TAACTACGAT ACGGGAGGGC TTACCATCTG GCCCCAGTGC TGCAATGATA CCGCGAGACC 4320
   CACGCTCACC GGCTCCAGAT TTATCAGCAA TAAACCAGCC AGCCGGAAGG GCCGAGCGCA 4380
   GAAGTGGTCC TGCAACTTTA TCCGCCTCCA TCCAGTCTAT TAATTGTTGC CGGGAAGCTA 4440
   GAGTAAGTAG TTCGCCAGTT AATAGTTTGC GCAACGTTGT TGCCATTGCT ACAGGCATCG 4500
   TGGTGTCACG CTCGTCGTTT GGTATGGCTT CATTCAGCTC CGGTTCCCAA CGATCAAGGC 4560
   GAGTTACATG ATCCCCCATG TTGTGCAAAA AAGCGGTTAG CTCCTTCGGT CCTCCGATCG 4620
   TTGTCAGAAG TAAGTTGGCC GCAGTGTTAT CACTCATGGT TATGGCAGCA CTGCATAATT 4680
   CTCTTACTGT CATGCCATCC GTAAGATGCT TTTCTGTGAC TGGTGAGTAC TCAACCAAGT 4740
   CATTCTGAGA ATAGTGTATG CGGCGACCGA GTTGCTCTTG CCCGGCGTCA ATACGGGATA 4800
   ATACCGCGCC ACATAGCAGA ACTTTAAAAG TGCTCATCAT TGGAAAACGT TCTTCGGGGC 4860
   GAAAACTCTC AAGGATCTTA CCGCTGTTGA GATCCAGTTC GATGTAACCC ACTCGTGCAC 4920
   CCAACTGATC TTCAGCATCT TTTACTTTCA CCAGCGTTTC TGGGTGAGCA AAAACAGGAA 4980
   GGCAAAATGC CGCAAAAAAG GGAATAAGGG CGACACGGAA ATGTTGAATA CTCATACTCT 5040
   TCCTTTTTCA ATATTATTGA AGCATTTATC AGGGTTATTG TCTCATGAGC GGATACATAT 5100
   TTGAATGTAT TTAGAAAAAT AAACAAATAG GGGTTCCGCG CACATTTCCC CGAAAAGTGC 5160
   CACCTGACGT CTAAGAAACC ATTATTATCA TGACATTAAC CTATAAAAAT AGGCGTATCA 5220
   CGAGGCCCCT TTCGTCTCGC GCGTTTCGGT GATGACGGTG AAAACCTCTG ACACATGCAG 5280
   CTCCCGGAGA CGGTCACAGC TTGTCTGTAA GCGGATGCCG GGAGCAGACA AGCCCGTCAG 5340
   GGCGCGTCAG CGGGTGTTGG CGGGTGTCGG GGCTGGCTTA ACTATGCGGC ATCAGAGCAG 5400
   ATTGTACTGA GAGTGCACCA TATGCGGTGT GAAATACCGC ACAGATGCGT AAGGAGAAAA 5460
   TACCGCATCA GGAAATTGTA AACGTTAATA TTTTGTTAAA ATTCGCGTTA AATTTTTGTT 5520
   AAATCAGCTC ATTTTTTAAC CAATAGGCCG AAATCGGCAA AATCCCTTAT AAATCAAAAG 5580
   AATAGACCGA GATAGGGTTG AGTGTTGTTC CAGTTTGGAA CAAGAGTCCA CTATTAAAGA 5640
   ACGTGGACTC CAACGTCAAA GGGCGAAAAA CCGTCTATCA GGGCGATGGC CCACTACGTG 5700
   AACCATCACC CTAATCAAGT TTTTTGGGGT CGAGGTGCCG TAAAGCACTA AATCGGAACC 5760
   CTAAAGGGAG CCCCCGATTT AGAGCTTGAC GGGGAAAGCC GGCGAACGTG GCGAGAAAGG 5820
   AAGGGAAGAA AGCGAAAGGA GCGGGCGCTA GGGCGCTGGC AAGTGTAGCG GTCACGCTGC 5880
   GCGTAACCAC CACACCCGCC GCGCTTAATG CGCCGCTACA GGGCGCGTCG CGCCATTCGC 5940
   CATTCAGGCT GCGCAACTGT TGGGAAGGGC GATCGGTGCG GGCCTCTTCG CTATTACGCC 6000
   AGCTGGCGAA AGGGGGATGT GCTGCAAGGC GATTAAGTTG GGTAACGCCA GGGTTTTCCC 6060
   AGTCACGACG TTGTAAAACG ACGGCCAGTG AATT 6094
(2) INFORMATIONS POUR LA SEQ ID NO: 4:
   (i) CARACTERISTIQUES DE LA SEQUENCE:
      (A) LONGUEUR: 36 paires de bases
      (B) TYPE: nucléotide
      (C) NOMBRE DE BRINS: simple
      (D) CONFIGURATION: linéaire
   (ii) TYPE DE MOLECULE: ADN (génomique)
   (ix) CARACTERISTIQUE:
      (A) NOM/CLE: SG52
   (xi) DESCRIPTION DE LA SEQUENCE: SEQ ID NO: 4:
   CTGGAAGATG GCGATCTCGA GATTCAGCAG GCCACT 36
(2) INFORMATIONS POUR LA SEQ ID NO: 5:
   (i) CARACTERISTIQUES DE LA SEQUENCE:
      (A) LONGUEUR: 37 paires de bases
      (B) TYPE: nucléotide
      (C) NOMBRE DE BRINS: simple
      (D) CONFIGURATION: linéaire
   (ii) TYPE DE MOLECULE: ADN (génomique)
   (ix) CARACTERISTIQUE:
      (A) NOM/CLE: SG53
   (xi) DESCRIPTION DE LA SEQUENCE: SEQ ID NO: 5:
   CTGTTTCATC AAAAGGGTAC CAGCCGTGGA GGGGCAT 37
(2) INFORMATIONS POUR LA SEQ ID NO: 6:
   (i) CARACTERISTIQUES DE LA SEQUENCE:
      (A) LONGUEUR: 24 paires de bases
      (B) TYPE: nucléotide
      (C) NOMBRE DE BRINS: simple
      (D) CONFIGURATION: linéaire
   (ii) TYPE DE MOLECULE: ADN (génomique)
   (ix) CARACTERISTIQUE:
      (A) NOM/CLE: oligonucléotide synthétique
   (xi) DESCRIPTION DE LA SEQUENCE: SEQ ID NO: 6:
   ATTATTATTA CTCGAGTGAT GATG 24
(2) INFORMATIONS POUR LA SEQ ID NO: 7:
   (i) CARACTERISTIQUES DE LA SEQUENCE:
      (A) LONGUEUR: 24 paires de bases
      (B) TYPE: nucléotide
      (C) NOMBRE DE BRINS: simple
      (D) CONFIGURATION: linéaire
   (ii) TYPE DE MOLECULE: ADN (génomique)
   (ix) CARACTERISTIQUE:
      (A) NOM/CLE: oligonucléotide synthétique
   (xi) DESCRIPTION DE LA SEQUENCE: SEQ ID NO: 7:
   ATCATCATCA TCCGAGTAAT AATA 24
(2) INFORMATIONS POUR LA SEQ ID NO: 8:
   (i) CARACTERISTIQUES DE LA SEQUENCE:
      (A) LONGUEUR: 14 paires de bases
      (B) TYPE: nucléotide
      (C) NOMBRE DE BRINS: simple
      (D) CONFIGURATION: linéaire
   (ii) TYPE DE MOLECULE: ADN (génomique)
   (ix) CARACTERISTIQUE:
      (A) NOM/CLE: SB211
   (xi) DESCRIPTION DE LA SEQUENCE: SEQ ID NO: 8:
   GGCAAACACT ATGG 14
(2) INFORMATIONS POUR LA SEQ ID NO: 9:
   (i) CARACTERISTIQUES DE LA SEQUENCE:
      (A) LONGUEUR: 17 paires de bases
      (B) TYPE: nucléotide
      (C) NOMBRE DE BRINS: simple
      (D) CONFIGURATION: linéaire
   (ii) TYPE DE MOLECULE: ADN (génomique)
   (ix) CARACTERISTIQUE:
      (A) NOM/CLE: PZ105
   (xi) DESCRIPTION DE LA SEQUENCE: SEQ ID NO: 9:
   TTGCGTACTG TCCTCTT                                                    17
(2) INFORMATIONS POUR LA SEQ ID NO: 10:
   (i) CARACTERISTIQUES DE LA SEQUENCE:
      (A) LONGUEUR: 20 paires de bases
      (B) TYPE: nucléotide
      (C) NOMBRE DE BRINS: simple
      (D) CONFIGURATION: linéaire
   (ii) TYPE DE MOLECULE: ADN (génomique)
   (ix) CARACTERISTIQUE:
      (A) NOM/CLE: oligonucléotide synthétique
   (xi) DESCRIPTION DE LA SEQUENCE: SEQ ID NO: 10:
   AAGAAGCCCT TGCAGCCCTC                                                 20
(2) INFORMATIONS POUR LA SEQ ID NO: 11:
   (i) CARACTERISTIQUES DE LA SEQUENCE:
      (A) LONGUEUR: 40 paires de bases
      (B) TYPE: nucléotide
      (C) NOMBRE DE BRINS: simple
      (D) CONFIGURATION: linéaire
   (ii) TYPE DE MOLECULE: ADN (génomique)
   (ix) CARACTERISTIQUE:
      (A) NOM/CLE: oligonucléotide synthétique
   (xi) DESCRIPTION DE LA SEQUENCE: SEQ ID NO: 11:
   AATTATAACT TCGTATAATG TATGCTATAC GAAGTTATTC                           40
(2) INFORMATIONS POUR LA SEQ ID NO: 12:
   (i) CARACTERISTIQUES DE LA SEQUENCE:
      (A) LONGUEUR: 20 paires de bases
      (B) TYPE: nucléotide
      (C) NOMBRE DE BRINS: simple
      (D) CONFIGURATION: linéaire
   (ii) TYPE DE MOLECULE: ADN (génomique)
   (ix) CARACTERISTIQUE:
      (A) NOM/CLE: amorce
   (xi) DESCRIPTION DE LA SEQUENCE: SEQ ID NO: 12:
   ATCCGAAAAG AAAACGTTGA 20
(2) INFORMATIONS POUR LA SEQ ID NO: 13:
   (i) CARACTERISTIQUES DE LA SEQUENCE:
      (A) LONGUEUR: 20 paires de bases
      (B) TYPE: nucléotide
      (C) NOMBRE DE BRINS: simple
      (D) CONFIGURATION: linéaire
   (ii) TYPE DE MOLECULE: ADN (génomique)
   (ix) CARACTERISTIQUE:
      (A) NOM/CLE: amorce
   (xi) DESCRIPTION DE LA SEQUENCE: SEQ ID NO: 13:
   ATCCAGGTTA CGGATATAGT 20
(2) INFORMATIONS POUR LA SEQ ID NO: 14:
   (i) CARACTERISTIQUES DE LA SEQUENCE:
      (A) LONGUEUR: 20 paires de bases
      (B) TYPE: nucléotide
      (C) NOMBRE DE BRINS: simple
      (D) CONFIGURATION: linéaire
   (ii) TYPE DE MOLECULE: ADN (génomique)
   (ix) CARACTERISTIQUE:
      (A) NOM/CLE: amorce
   (xi) DESCRIPTION DE LA SEQUENCE: SEQ ID NO: 14:
   GGTTCTCCGG CCGCTTGGGT 20
(2) INFORMATIONS POUR LA SEQ ID NO: 15:
   (i) CARACTERISTIQUES DE LA SEQUENCE:
      (A) LONGUEUR: 20 paires de bases
      (B) TYPE: nucléotide
      (C) NOMBRE DE BRINS: simple
      (D) CONFIGURATION: linéaire
   (ii) TYPE DE MOLECULE: ADN (génomique)
   (ix) CARACTERISTIQUE:
      (A) NOM/CLE: amorce
   (xi) DESCRIPTION DE LA SEQUENCE: SEQ ID NO: 15:
   GAAGGCGATG CGCTGCGAAT 20
(2) INFORMATIONS POUR LA SEQ ID NO: 16:
   (i) CARACTERISTIQUES DE LA SEQUENCE:
      (A) LONGUEUR: 20 paires de bases
      (B) TYPE: nucléotide
      (C) NOMBRE DE BRINS: simple
      (D) CONFIGURATION: linéaire
   (ii) TYPE DE MOLECULE: ADN (génomique)
   (ix) CARACTERISTIQUE:
      (A) NOM/CLE: amorce A
   (xi) DESCRIPTION DE LA SEQUENCE: SEQ ID NO: 16:
   CAAGGAGCCT CCTTTCTCTA 20
(2) INFORMATIONS POUR LA SEQ ID NO: 17:
   (i) CARACTERISTIQUES DE LA SEQUENCE:
      (A) LONGUEUR: 20 paires de bases
      (B) TYPE: nucléotide
      (C) NOMBRE DE BRINS: simple
      (D) CONFIGURATION: linéaire
   (ii) TYPE DE MOLECULE: ADN (génomique)
   (ix) CARACTERISTIQUE:
      (A) NOM/CLE: amorce B
   (xi) DESCRIPTION DE LA SEQUENCE: SEQ ID NO: 17:
   CCTGCTCTAC CTGGTGACTT                                                    20